# EUROPÄISCHE PATENTSCHRIFT

(11) **EP 3 624 960 B1**
(45) Veröffentlichungstag und Bekanntmachung des Hinweises auf die Patenterteilung: **13.03.2024**
(21) Anmeldenummer: 18733163.2
(22) Anmeldetag: 15.05.2018
(51) Int. Cl.: B09B 3/00, C05F 17/00, C02F 11/04

(54) **VERFAHREN ZUR ENTFERNUNG VON ORGANISCHEN UND ANORGANISCHEN SCHADSTOFFEN AUS ABFÄLLEN MITTELS NASSMECHANISCHER TRENNUNG**
METHOD FOR REMOVING ORGANIC AND INORGANIC HARMFUL SUBSTANCES FROM WASTE BY MEANS OF WET MECHANICAL SEPARATION
PROCÉDÉ POUR ÉLIMINER DES SUBSTANCES NOCIVES ORGANIQUES OU INORGANIQUES DE DÉCHETS AU MOYEN D'UNE SÉPARATION GRAVIMÉTRIQUE

(30) Priorität: 15.05.2017 DE 102017110474
(43) Veröffentlichungstag der Anmeldung: 25.03.2020
(73) Patentinhaber: EcoEnergy Gesellschaft für Energie- und Umwelttechnik mbH, 37075 Göttingen (DE)
(72) Erfinder: SCHU, Reinhard, 37075 Göttingen (DE); SCHU, Kirsten, 37075 Göttingen (DE)
(74) Vertreter: RGTH
(86) Internationale Anmeldenummer: PCT/DE2018/100462
(87) Internationale Veröffentlichungsnummer: WO 2018/210379

(56) Entgegenhaltungen:
- WO-A1-2005/051547
- WO-A1-2010/118730
- DE-A1- 4 117 515
- DE-A1- 4 322 100

## Beschreibung

### Einleitung

Über Abfall und Abwasser gelangen organische und anorganische Schadstoffe in die Umwelt. Die Stoffe stammen aus Nahrungs- und Genussmitteln, Kleidung, Lebensmittelzusätzen, Kosmetika, pharmazeutischen Produkten, Reinigungs- und Waschmitteln, Kunststoffen sowie diverse Chemikalien.

Abfallpolitisches Ziel ist es, natürliche Ressourcen zu schonen und den Schutz der Umwelt sicherzustellen. Das Wirtschaftswachstum und die mit der Abfallerzeugung verbundenen Auswirkungen auf die Umwelt müssen entkoppelt werden. Bevölkerungswachstum und Ressourcenverknappung zwingen die Produzenten zum Umdenken. Besonders kritisch ist die Ressourcenknappheit bei dem nicht ersetzbaren Element Phosphor, dessen Verfügbarkeit die Grundlage allen Lebens auf der Erde ist. Phosphor wurde, als einziges essentielles Element, 2014 von der Europäischen Union in die Liste der kritischen Rohstoffe aufgenommen. Im ersten Schritt soll der Einsatz von Phosphat in der Produktion möglichst vermeiden werden. Für die Industrie bedeutet dies einschneidende Veränderungen bei der Produktion, vergleichbar den Auswirkungen der Energiewende auf die Energiekonzerne. Der Peak Phosphor, die maximale globale Phosphatproduktion, wird von Experten bereits für das Jahr 2030 und früher prognostiziert.

Phosphor kann aus pflanzlichen und tierischen Reststoffen, Siedlungsabfällen und Abwasser zurückgewonnen werden. Insbesondere die Verwertung von Klärschlamm, aber auch von Kompost, ist begrenzt durch die Belastung mit Schwermetallen und organischen Schadstoffen. In Industrieländern wie Deutschland kommt erschwerend hinzu, dass aufgrund der separaten Sammlung von Bioabfall und der damit verbundenen Kompostproduktion, der hohen Anzahl von landwirtschaftlichen Biogasanlagen und landwirtschaftliche Reststoffe bereits zu viel Stickstoff und Phosphor regional konzentriert anfällt.

Die Verwendung vieler organischer Schadstoffe wie Dioxine, Furane, DDT, PCB und PCP wurden über die Stockholm Convention on Persistent Organic Pollutants und andere Vereinbarungen schon vor 10 - 20 Jahren international verboten. Die Konzentration dieser organischen Schadstoffe hat seitdem im Klärschlamm und Kompost kontinuierlich abgenommen (Quelle: Klärschlamm und Boden - Eintrag von Spurenstoffen auf landwirtschaftlich genützte Böden; Bericht Bericht UI-05/2016; Hrsg.: Amt der Vorarlberger Landesregierung, Römerstraße 15, 6901 Bregenz, Österreich). In Westeuropa konnten auch die Schwermetallkonzentration im Klärschlamm durch Verordnungen und separate Reinigung von gewerblichem Abwasser stark reduziert werden.

Das deutsche Bundesumweltministerium (BMU) und das Bundesministerium für Verbraucherschutz, Ernährung und Landwirtschaft (BMVEL) haben auf Basis einer Studie des Umweltbundesamtes am 28.08.2002 strengere Vorgaben für einheitliche Schwermetallgrenzwerte für Sekundärdüngemittel für eine nachhaltige landwirtschaftliche Nutzung vorgestellt. Da diese Schwermetallgrenzwerte nach dem damaligen Stand der Technik einem Verbot der landwirtschaftlichen Nutzung von Bioabfallkompost in Deutschland gleichgekommen wären, kam der Entwurf nicht zur Anwendung.

Auf Empfehlung des deutschen Umweltbundesamtes wurde im Dezember 2002 ein Forschungsprojekt zur Schwermetallentfrachtung aus Restabfall und Bioabfall von der Deutschen Bundesstiftung Umwelt (DBU) gefördert und von EcoEnergy Gesellschaft für Energie- und Umwelttechnik GmbH, Göttingen, Deutschland von 2004 bis 2009 durchgeführt.

Das im Forschungsprojekt im Pilotmaßstab erprobte Verfahren besteht aus einer mehrstufigen kontinuierlichen nassmechanischen Abfallwäsche mit Schwerstoffabscheidung und Nasssiebung (Patent EP1687093B1 und US7469846B2, Priorität 2003) sowie einer Abscheidung von Kunststoffen durch thermische Zelllyse (Patent EP2059319B, US020100006515A1, JP5610767B2, Priorität 2006). Die meisten organischen Schadstoffe sowie Schwermetalle befinden sich in der Kunststofffraktion, in der diese Schadstoffe fast ausschließlich wasserunlöslich eingebunden sind. Eine weitere Schadstoffsenke ist die Feinfraktion < 100 µm. Es konnte nachgewiesen werden, dass auch die Organik-Fraktion 0,1 mm bis 8 mm aus Restabfall die Düngemittelgrenzwerte für Schwermetalle und organische Schadstoffe einhalten konnte, soweit die Kunststofffraktion und Feinmineralfraktion effektiv abgetrennt wurden. Bei Einsatz von separat gesammeltem Bioabfall konnten die am 28.08.2002 vom deutschen Umweltministerium geforderten nachhaltigen Vorsorgegrenzwerte weit unterschritten werden.

Im Forschungsprojektes (DBU AZ- 08449/01) wurde vorgeschlagen, die Feinfraktion < 100 µm zu vergären, um Schwermetalle und Phosphat aufzukonzentrieren und - analog zu Klärschlamm - nach einer Vergärung den Klärschlamm abzuscheiden. Eine technische Lösung zur Trennung von Schwermetallen aus der Feinfraktion vor einer biologischen Behandlung, wie in der vorliegenden Erfindung, gab es zum damaligen Zeitpunkt noch nicht.

Im Anschluss an das Forschungsprojekt DBU AZ-08449/01 wurde in der Schweiz auf der KBA Hard, Schaffhausen, eine Speisereste-, Bio- und Restabfallvergärungs- sowie Klärschlammtrocknungsanlage nach dem patentierten Verfahren gebaut und erfolgreich in Betrieb genommen (Quelle: 18.12.2012, Stadt Schaffhausen, KBA Hard: Energiegewinnung aus Abfall - ein Schritt in die Zukunft ist geglückt*).* Die Anlage war Teil eines Verbundvorhabens zum Nachweis der landwirtschaftlichen Verwertung von Aschen aus der Verbrennung von Gärresten aus diversen Abfallfraktionen. In einer Schlammverbrennung sollten Schlämme aus der Vergärung von tierischen Abfällen, Speiseresten, Bio- und Restabfall sowie Klärschlamm gemeinsam verbrannt werden. Mit dem thermochemischen Verfahren der Ash Dec Umwelt AG sollte aus der Asche ein Recyclingdünger gewonnen werden. Bereits in der Versuchsanlage der Ash Dec Umwelt AG hatte sich gezeigt, dass es unlösbare Probleme gab, so dass die großtechnische Anlage nicht wie geplant, 2012/2013 realisiert werden konnte. WO2005051547 offenbart ein Verfahren zur nassmechanischen Behandlung eines Stoffgemisches, insbesondere von Abfall jeder Art, bestehend aus Inertstoffen, Wasser sowie organischen Stoffen mit einem wasserlöslichen und biologisch umsetzbaren Anteil, wobei Wasser als Löse-, Wasch- und Trennmittel eingesetzt wird.

Das erfindungsgemäße Verfahren nutzt die Erkenntnis, dass in biologisch behandelten Abfällen, wie Gärresten oder Klärschlamm, die Schadstoffe vorwiegend in der neu aufgebauten Biomasse, genauer in den Exopolysacchariden (gewöhnlich als Biofilm oder Bioschleim bezeichnet), gebunden sind. Dieser Effekt wird bei der Abwasserbehandlung nach dem Stand der Technik genutzt, um die Schadstoffe in den Klärschlamm zu überführen und das Abwasser zu reinigen.

Mit dem erfindungsgemäßen Verfahren werden die Abfälle bzw. Feststoffe aus dem Abwasser bereits vor einer biologischen Behandlung und damit vor einer Umlagerung der Schadstoffe behandelt. Schwermetalle können am effektivsten mit Schwefelsäure und anschließender sulfidischer Fällung aus Abfällen ausgewaschen werden. Frische Abfälle und frisch abgesiebter Feststoff aus Abwasser sind meist sauer und benötigen nur wenig Schwefelsäure für die Auswaschung von Schermetallen.

Biologisch behandelte Abfälle, wie Gärreste oder Klärschlamm, sind hingegen meist alkalisch und gepuffert, so dass große Mengen an Schwefelsäure für die Auswaschung erforderlich sind. Eine Vergärung nach der chemischen Wäsche mit Schwefelsäure wird, ist aufgrund der großen erforderlichen Säuremengen und des geringen Anteils leicht biologisch abbaubarer Stoffe nur unter Zugabe von zusätzlichen Oxidantien, wie zum Beispiel Methanol, möglich. Nur bei geringsten Substratkonzentrationen mit einem Verhältnis von CSB zu Sulfat von kleiner als 1,7 können Sulfatreduzierer über Methanogene dominieren (Quelle: Preuß, Volker; 2005: Konkurrenz zwischen Methanogenen und Desulfurikanten bei der biochemischen Entsäuerung von Bergbauwässern bei Verwendung von Methanol als C- und Energiequelle. In: Merkel, Schaeben, Wolkersdorfer, Hasche (Hrsg.) Behandlungstechnologien für bergbaubeeinflusste Wässer; TU Bergalkademie Freiberg - Tagungsband; 56. Hüttenmännischer Tag).

Daher werden mit dem erfindungsgemäßen Verfahren nur Abfälle behandelt, die über 20% biogenen Anteil aufweisen, welcher überwiegend leicht biologisch anaerob abbaubar ist.

Seit 75 Jahren wird mit Verfahren der mechanischen, chemischen und thermischen Klärschlammdesintegration versucht, die Exopolysaccharidhülle aufzubrechen, um Schadstoffe oder Phosphor abzutrennen oder auch um die Entwässerung zu verbessern. Effektive thermische Verfahren sind sehr aufwendig, bilden organische Schadstoffe, wie zum Beispiel Dioxin, und führen bei Kläranlagen zu einer Schwermetallrückbelastung des Abwassers, zum Beispiel mit Quecksilber (Quellen: Klärschlammdesintegration - Überblick über verschiedene Verfahren, 2004, K. Nickel, U. Neis, TU Hamburg-Harburg, URL http://www.ultrawaves.de/unternehmen/downloads/klaerschlammdesintegration und Thermische Schlammkonditionierung nach dem Porteous-Verfahren: Bilanzierung der PCDD/F, PCB und Schwermetalle, UWSF-Z.Umweltchem. Okotox. 5 (5) 1993).

Thermische Konditionierungsverfahren mit Temperaturen über 160°C wurden mit der Einführung der Dioxingrenzwerte für die landwirtschaftliche Klärschlammverwertung 1992 wieder aufgegeben. Leider sind die meisten Betreiber sehr vorsichtig mit der Veröffentlichung von Meldungen über "Dioxin", so dass sich wieder neue Hochtemperaturkonditionierungsverfahren und Biokohleverfahren etabliert haben, die ebenfalls Probleme mit der Dioxinentstehung, gerade bei der Verwendung von Klärschlamm, haben. Klärschlamm enthält viel Kupferoxid, das aus den Trinkwasserleitungen stammt. Das Kupferoxid ist ein sehr guter Katalysator für die Dioxinentstehung durch die De Novo-Synthese auch in wässrigen Systemen. Für Abfälle mit hohen Schwermetallgehalten sind daher thermische Verfahren mit Temperaturen > 120°C zu vermeiden.

Chemische Verfahren benötigen sehr viel Säure, da sich durch die vorherige biologische Behandlung ein alkalisches Puffersystem eingestellt hat.

Speziell in Kläranlagen kann durch die biologische Behandlung verfügbar gewordenes Phosphat durch Kristallisation z.B. als Magnesium-Ammonium-Phosphat ausgeschleust und als Dünger verwendet werden. Mittels Unterstützung durch thermische Konditionierungsverfahren kann der gewonnene Anteil des Phosphates aus dem Abwasser theoretisch von ca. 50 % auf 70 % erhöht werden. Unter realen Bedingungen wird jedoch meist nur eine Abscheideleistung für Phosphor von unter 50% erreicht.

Auch wenn durch die thermische Konditionierung der überwiegende Anteil des Phosphors bereits im Kläranlagenprozess rückgewonnen werden kann, reduziert sich zwar die Klärschlammmenge aber gleichzeitig erhöht sich auch die spezifische Belastung mit Schwermetallen, bezogen auf die Trockensubstanz und bezogen auf die im Klärschlamm verbleibende Restphosphatmenge. Nach aktuellem Stand der Technik soll nach der Klärschlammverordnung vom 27. September 2017 in Deutschland Klärschlamm aufgrund seiner Belastung mit organischen Schadstoffen aber auch aufgrund der erhöhten Schwermetallbelastung thermisch behandelt werden und das Phosphat aus den Aschen zurückgewonnen werden.

Für die Rückgewinnung des Phosphates aus den Klärschlammaschen werden mehr als 30 verschiedene Verfahren genannt, von denen bisher kein Verfahren wirtschaftlich ist bzw. erprobt ist. In der aktuellen Verfahrensübersicht "Statuspapier Phosphatrückgewinnung - Statuspapier der ProcessNet-Fachgruppe Rohstoffe" vom Oktober 2017 der DECHEMA Gesellschaft für Chemische Technik und Biotechnologie e.V. werden ca. 70 Verfahren genannt, von denen nur wenige kommerziell zur Phosphorrückgewinnung als Magnesiumammoniumphosphat im Klärwerksprozess betrieben werden. Keines der genannten Verfahren wird kommerziell zur Rückgewinnung von Phosphor aus der thermischen Klärschlammbehandlung eingesetzt. Fast alle der genannten Verfahren werden nicht einmal mehr vertrieben.

So wird weder das Ash Dec-Verfahren (mit chlorierendem Rösten der Aschen zum Austreiben der Schwermetalle als Schwermetallchloride) weiter angeboten noch das Eberhard Verfahren (auch genannt EBIPHOS), Patent Nr. CH697083, da dieses Verfahren nicht wirtschaftlich zu betreiben war.

Wenn aber eine Phosphatrückgewinnung aus den Klärschlammaschen langfristig vorgeschrieben werden soll, da die organischen und anorganischen Schadstoffe nicht ausreichend reduziert werden können, ist es verfahrenstechnisch sinnvoll, stattdessen den Klärschlamm entsprechend zu reinigen und direkt landwirtschaftlich zu verwerten. Mit dieser Verfahrensidee wurde 2000 das Seaborne-Verfahren entwickelt und in einem Forschungsprojekt, gefördert durch das Land Niedersachsen, von 2008 bis 2010 großtechnisch betrieben und getestet (Quelle: Wissenschaftliche Begleitung der großtechnischen Anwendung der Seaborne-Technologie auf der Kläranlage Gifhorn, Abschlussbericht September 2012, www.asg-gifthorn.de/docs/abschlussbericht seaborne technologie gifthorn.pdf ) Das Seaborne-Verfahren besteht aus einer sauren Wäsche der Klärschlämme mit Schwefelsäure und einer anschließenden sulfidischen Fällung der gelösten Schwermetalle aus dem Waschwasser. Das Verfahren wurde nicht weiterverfolgt, da der Schwefelsäureverbrauch sehr hoch war und die Auswaschung der Schwermetalle aufgrund der Exopolysaccharide nicht ausreichend erfolgte.

Bei der thermischen Aufbereitung - durch Verbrennung und Nachbehandlung der Asche - können jedoch Dioxine entstehen, so wie z.B. bei dem in der Schweiz durch Holcim (AT 405 191 B, Priorität 08.02.1996) und bei dem Paul-Scherrer-Institut (Dissertation ETH No. 14653 von 2002, WO2001054800A1, Anmeldung 25.01.2001) entwickelten chlorierenden Röstverfahren, dem Verfahrensprinzip des Ash Dec-Verfahren. Die Verfahrensentwicklung basierte auf einem Patent (DE000000444612A), das 1925 für die Gewinnung von Kupferchloriden aus kupferarmen Schiefergestein, speziell für die Herman Göring Werke in Marsberg angemeldet worden war. Von 1937 bis 1945 wurde das Verfahren betrieben und es wurden ca. 5 Mio. Tonnen dioxinbelastete Schlacke produziert. Die Schlacke wurde von 1955 bis 1975 als Marsberger Kieselrot für Sport- und Spielplätze eingesetzt. Erst 1991 wurde bekannt, dass das Marsberger Kieselrot große Mengen Dioxine enthält. Die Weiterentwicklung des chlorierenden Röstens mit dem Ash Dec-Verfahren wurde eingestellt.

Die Firma Ash Dec Umwelt AG hatte allerdings veröffentlicht, dass der Dünger 2006 in Österreich und 2008 in Ungarn und Deutschland als Qualitätsdünger zugelassen und aus dem Abfallregime entlassen wurde. Die Firma hat angegeben, dass durch das Ash Dec Verfahren kein Dioxin produziert wurde und der Gehalt an organischen Schadstoffen vernachlässigbar sei.

Im Rahmen einer aktuellen Schweizer Studie zur Festlegung von Grenzwerten für mineralische Recyclingdünger wurde jedoch in einem durch chlorierendes Rösten, analog dem AshDec Verfahren, erzeugten Recyclingdünger die höchste Konzentration an PCDD/F gemessen. Es wird in der Studie darauf hingewiesen, dass aufgrund der Gefahr einer Entstehung von Dioxin durch "de novo" Synthese bei Vorhandensein von Chlor, kritisch zu betrachten ist, inwieweit die thermischen Verfahren eine geringe Dioxinbelastung im Dünger sicher einhalten können, vor allem wenn die erforderlichen hohen Temperaturen der Nachverbrennung zur Dioxinzerstörung nicht erreicht werden. Auch bei der Pyrolyse können PCDD/F entstehen. Grundsätzlich können sich PCDD/F thermo-chemisch sowohl aus Vorläuferverbindungen wie Chlorbenzolen, - phenolen oder PCB bilden, als auch "de novo" aus kleineren Molekülen und Chlorid entstehen. Höhere Konzentrationen von Vorläuferverbindungen, aber auch Chlorid, können die Bildung und das Vorkommen von PCDD/F begünstigen. (Quelle: Entwicklung agronomischer und ökologischer Anforderungen an die Mindestqualität von Mineralischen Recyclingdüngern (MinRec); Weggler K., Richner W., Reiser R., Bucheli T., Bürge D. und Mayer J.; 2017; Herausgeber: Agroscope, Reckenholzstrasse 191, 8046 Zürich, Schweiz)

Im Versuchsbetrieb des Ash Dec Verfahrens 2008 wurde der Dünger für die Markterprobung mit einem im Gleichstrom betriebenen Drehrohrofen hergestellt. In diesem Versuchsbetrieb wurden Pellets eingesetzt, um eine zu hohe Flugaschefracht zu vermeiden. Um die Stabilität der Pellets zu gewährleisten, wurden diese langsam aufgeheizt und langsam abgekühlt. Die Beheizungstemperaturen mussten nach Angaben der Betreiber auf < 1.000 °C gehalten werden, da ansonsten die Standzeit der Ausmauerung einen kontinuierlichen Betrieb verhindert hätte. Nach eigenen Berechnungen können mit dieser Betriebsweise und dem verwendeten Drehrohrofensystem die Temperaturen der Pellets im Austrag nicht wesentlich eine Temperatur von 600 °C überschritten haben. Dies ergeben auch Auswertungen des Film- und Bildmaterials von der Bundesanstalt für Materialforschung und -prüfung (BAM) und Ash Dec Umwelt AG.

Nach eigenen Berechnungen können mit diesem Verfahren die Grenzwerte für Dioxin, die für einen Dünger in Deutschland, Ungarn und der Schweiz vorgeschrieben sind, nicht eingehalten werden. Der Betreiber gibt an, das Verfahren trotz voller Auftragsbücher in der Größenordnung von 1 Milliarde Euro Umsatz und Finanzierungsbestätigung mehrerer Projekte aufgegeben zu haben, da die Anlage nachts schwer zu betreiben sei und aufgrund akuter Schwierigkeiten mit dem Absatz von Recyclingdünger (Quelle: Jahresbericht Ash Dec Umwelt AG, 2009). Ash Dec Umwelt AG meldete 2010 Insolvenz an und wurde liquidiert.

Die Umstellung des chlorierenden Röstverfahrens auf unterstöchiometrische Fahrweise zur Vermeidung der Entstehung von Dioxin oder der Ersatz der eingesetzten Chlor-Additive durch Schwefel oder Carbonat führte nicht zur gewünschten Schwermetallentfrachtung oder die Pflanzenverfügbarkeit des Phosphors war nicht zufriedenstellend oder Aufwand und damit Kosten waren zu hoch.

Sowohl die Weiterentwicklung des PECK- (PSI, Eberhard Recycling AG, CT Umwelt AG, Küpat AG) bzw. CT-Fluapur -Verfahrens mit dem chlorierenden Röst-Verfahren in der Weiterentwicklung des Ash Dec-Verfahrens und das EBIMIN-Verfahren als auch die Trockenentaschung, die nach einer Mediationsphase in Hinwil mit 2 Jahren Verzögerung in Betrieb genommen werden konnte, wie der langjährige Entwicklungsleiter der Eberhard Recycling AG, Dr. Markus Franz, berichtet, wird ebenfalls wegen der Dioxinentstehung nicht weiterverfolgt.

Hohe Anteile von Elektronikschrott in der Müllverbrennungsanlage Hinwil bei Zürich verursachen neben hohen Chlorgehalten aus dem PVC auch hohe Kupfergehalte, die die Dioxinentstehung bei langsamer Abkühlung der Aschen katalysieren. (Quelle: Dr. Ing. Markus Franz, Juni 2014, Nutec Engineering AG, "KVA-Schlackenaufbereitung in der Schweiz - Von den Anfängen bis heute" und Dr. Ing. Markus Franz, September 2016, KBA Hard, Schaffhausen, "Geschichte der Schlackenaufbereitung" und: "11. September 2013 - Schachzug von Eberhard - Wir bauen kein Ebimin", http://www.cleano.ch/wordpress/?p=1558 )

Aber auch die Verwertung der Gärreste aus der KBA Hard, die mit dem Ash Dec-Verfahren vorgesehen war, konnte aufgrund der Erfahrung mit der Bildung von Dioxin nicht weitergeführt werden. Damit war auch die Betriebsgenehmigung und die Subvention des Biogases für die KBA Hard, zumindest für Hausmüll und nicht hygienisierten Bioabfall, ohne die Möglichkeit der landwirtschaftlichen Verwertung, nicht mehr gegeben. Die Anlage wurde dann auf Abfälle umgestellt, die auch ohne das nachgeschaltete Ash Dec-Verfahren landwirtschaftlich verwertbar sind (Quelle: 11.09.2013, srf, "KBA Hard: «Die Anlage kann nie erfolgreich betrieben werden» Zitat: ... *ein neuartiges Verbrennungsverfahren war geplant. Im Frühling 2013 sollte die neue Anlage fertig sein. «Die Anlage, so wie sie heute konzipiert ist, wird weder heute noch in Zukunft technisch oder betriebswirtschaftlich erfolgreich betrieben werden können», steht laut Mitteilung der Stadt im Bericht. Die Stadt plant deshalb eine «teilweise strategische Neuausrichtung» "* Quelle: 11.09.2013: Homepage der Stadt Schaffhausen: "KBA Hard: Fakten liegen auf dem Tisch" - Gemäss Experten lässt sich die Anlage nach dem ursprünglichen Konzept nicht erfolgreich realisieren.*")*.

Die thermochemische Erzeugung von Recyclingdünger aus Klärschlammasche muss drei Düngekriterien erfüllen: Entfrachtung von Schwermetallen, Vermeidung einer "de novo" Synthese von organischen Schadstoffen, vor allem Dioxin, und die Verbesserung der schlechten Pflanzenverfügbarkeit von Phosphat. Es existiert bisher kein Verfahren, das alle drei Anforderungen technisch und wirtschaftlich erfüllt, da verfahrensbedingt die Erreichung aller drei Ziele gleichzeitig nicht bzw. nicht mit vertretbarem Aufwand möglich ist.

Zudem wird durch die Verbrennung des Klärschlamms der für die Bodenfruchtbarkeit wesentliche Anteil der Tonminerale thermisch zerstört und auch die für die nachhaltige Phosphatverfügbarkeit wichtigen Huminstoffe und der Humusanteil fehlen vollständig. Auch wird durch die thermische Behandlung der Stickstoff vollständig als Stickoxid und Stickstoffgas ausgetrieben und geht der Landwirtschaft verloren.

Mit dem erfindungsgemäßen Verfahren hingegen, werden alle Nährstoffe und essentielle mineralische Bodenbestandteile wieder in die Landwirtschaft zurückgeführt, wobei organische Schadstoffe durch eine Kombination aus niederthermischer und biologischer Behandlung zerstört werden und die Schwermetalle durch saure nassmechanische Trennung abgeschieden werden.

### Erreichung der Produktqualitäten aus Abfällen durch das erfindungsgemäße Verfahren

Die Qualität der mit dem erfindungsgemäßen Verfahren erzeugten Produkte wird auch ohne separate Bioabfall- und Verpackungsabfall-Erfassung mittels saurer und heißer Auswaschung von Schwermetallen sowie Abscheidung von Störstoffen durch thermomechanische Zelllyse erreicht.

Die Abscheidung von sauberem verwertbarem Sand aus Abfällen bis hinunter zu einer Korngröße von 100 µm ist bekannt, siehe Nassmechanisches Trennverfahren Patent EP1687093B1 und US7469846B2. Nach dem Patent DE 196 17 501 C2 wurde eine Großanlage zur Baggergutaufbereitung aus dem Hamburger Hafen (METHA) für die Abscheidung des Schluffanteiles < 100 µm bis 20 µm 1996 erfolgreich nachgerüstet. Im Rahmen eines Förderprojektes der GKSS-Forschungszentrum Geesthacht GmbH, gefördert durch die Deutsche Bundesstiftung Umwelt, Aktenzeichen AZ 07995, wurden von 1996 bis 1999 umfangreiche Versuche zum Patent DE 197 19 034 C2 durchgeführt. Um eine verwertbare Mineralfraktion der Größen 5 µm bis 100 µm abzuscheiden, wurde eine Mineralstoffsuspension mittels Kavitationstechnik vorbehandelt. Bei der Kavitation werden lokal durch Unterdruck mittels Lochplatten Dampfblasen erzeugt, die beim Implodieren die Schadstoffe von den Schluffpartikeln reinigen sollen. Ein Kavitationseffekt kann ebenfalls durch Ultraschall erzeugt werden. Damit die Schadstoffe - so war die Hypothese - sich nicht direkt nach der Kavitationsbehandlung noch vor der mechanischen Trennung wieder koagulieren, wurden Oxidations- und Trennmittel zugegeben.

Es hat sich gezeigt, dass der mechanische Aufwand zur Trennung sehr hoch ist und sich die Tonmineralien zusammen mit der organischen Fraktion und dem Schluff schnell wieder zusammenfinden. Es hat sich erwiesen, dass sich die Schwermetallbelastung im Wesentlichen nicht an der organischen Fraktion, sondern an den Tonmineralien < 5 µm befindet. Dies gilt jedoch nicht für biologisch behandelte Abfälle bzw. Suspensionen, bei denen fast zwei Drittel der organischen Masse aus extrazellulären Polymeren Substanzen besteht, die vorwiegend negativ geladen sind und mit Schwermetallen säurestabile und weitgehend temperaturstabile Komplexe bilden. Tonminerale mit sehr großer äußerer und innerer Oberfläche, sind negativ geladen und fungieren als Kationenaustauscher (u.a. für Schwermetalle) im Boden und binden positiv geladene Nährstoffe wie Natrium, Kalium, Magnesium, Kalzium und Ammonium sowie Schwermetalle und seltene Erden. Diese Tonminerale können durch Säurebehandlung bei pH-Werten von kleiner 2 und erhöhter Temperatur von ca. 50 - 70°C gereinigt werden. Tonminerale sind nicht nur der "Schwamm für Schwermetalle" im Boden, sondern sind maßgeblich für das Bodengefüge durch Ton-Humus-, Ton-Ton- und Ton-Schluff-Komplexe verantwortlich. Diese Komplexe werden durch Kalziumbrücken gebildet und haben eine krümelige Struktur. Bei einer Versauerung wird Kalzium herausgelöst, die krümeligen Strukturen und damit das Bodengefüge zerstört und der Boden verschlammt. Genau dieser Zustand des Verschlämmens, der im natürlichen Boden vermieden werden soll, wird bei der Reinigung der Feinfraktion des erfindungsgemäßen Verfahrens aus Abfällen benötigt. Ab pH-Werten von kleiner 5,5 werden die Kalziumbrücken zerstört und eine Trennung von Schluff, Ton und Organik wird ohne Einbringung von hoher mechanischer Energie ermöglicht. Organik, Ton und Schluff liegen trennfähig vor. Aufwendige mechanische Energie mit entsprechendem Verschließ wie beim Kavitationsverfahren oder Prallplattenverfahren sind nur in sehr geringem Umfang erforderlich. Bei dem erfindungsgemäßen Verfahren wird daher ein pH-Wert von 4,5 bis 5,5, bevorzugt 5, eingestellt.

Die organischen Belastungen, die nicht durch Säurebehandlung alleine gereinigt werden können, sind vorrangig in der organischen Fraktion enthalten. Organische Belastungen können heute, nachdem alle bekannten persistenten organischen Schadstoffe (POP *persistent organic pollutants)* in der Europäischen Union verboten wurden, durch biologische Behandlungsverfahren gereinigt werden.

### Grundlagen der mehrstufigen technischen Konzentration von Tonmineralien

Bekannt sind Verfahren zur Trennung von Steinen, Kies und Sand aus Gemischen mit Organik durch gravimetrische Trennung mit Wasser und Luft. Die Trennung von Gemischen mit Korngrößen kleiner als 100 µm erfolgt nach dem Stand der Technik durch gravimetrische Schichtströmungsverfahren bzw. Filmdichtesortierung auf Sortierspiralen. Dies ist eine der ältesten und bis zum heutigen Tag eine der wirtschaftlichsten Methoden in der Mineralienaufbereitung. Erfunden wurden Sortierspiralen von Hundt 1863. Erstmals eingesetzt wurden Sortierspiralen 1941 von Humphreys Gold Corporation (Humphrey-Spiral) zur Goldgewinnung.

Heute wird grundsätzlich zwischen Kohle-Spiralen und Erz-Spiralen unterschieden, der Unterschied liegt vorrangig im Gefälle der Spiralen. Das Gefälle kann genau auf die Einsatzmaterialien angepasst werden.

Erst seit etwa 1990 werden Sortierspiralen im Umweltbereich in der Bodenaufbereitung eingesetzt, seit 1996 auch in der Hafenschlick- und der Straßenkehricht-Aufbereitung eingesetzt. Für die Bio- und Restabfallaufbereitung wurden Sortierspiralen erstmalig 2004 von der Firma EcoEnergy eingesetzt (Nassmechanisches Trennverfahren Patent EP1687093B1 und US7469846B2).

Die Sortierspirale wird bei hohem Anteil an Leichtgut und gut suspendierbaren Feststoffen mit geringem Anteil an Schwergut sowie großen Dichteunterschieden eingesetzt. Die Aufgabekorngröße sollte bei Kohlespiralen zwischen 0,1 bis 2 mm liegen. Die Durchsatzleistung pro Start beträgt ca. 2 - 3 t/h Feststoff bzw. bei einer Trübedichte von 15% - 40% ca. 8 - 12 m³/h Suspensionsdurchsatz.

Durch das erfindungsgemäße Verfahren werden durch die heiße chemische Wäsche die Schadstoffe in das Kreislaufwasser < 100 µm überführt. Durch Kavitationstechnik, ausgeführt mit Ultraschall, werden die an der Fraktion < 100 µm anhaftenden Schadstoffe mechanisch abgereinigt und die Partikel bis größer 5 µm als schadstoffarme Schlufffraktion abgetrennt. Durch die saure Wäsche ist der Schluff kalkarm und kann als Füller im Asphalt eingesetzt werden. Die Tonmineralien < 5 µm werden über Wendelscheider aufkonzentriert und in Rührbehälter mit Säure bei pH-Werten von 2 - 3,5 nachbehandelt. Danach werden die Tonmineralien < 5 µm nach Zugabe von Flockungshilfsmittel gefiltert und entwässert. Hierzu wird je nach Flockungshilfsmittel eine pH-Werterhöhung auf bis zu pH-Wert 5 erforderlich sein. Die gelösten Schwermetalle, die bis pH-Wert 5 gelöst bleiben und vom Flockungshilfsmittel nicht erfasst werden, befinden sich im Filtrat. Die Schwermetalle werden sulfidisch durch Einleitung von schwefelwasserstoffhaltigem Biogas oder durch Zugabe von Natriumsufid gefällt. Mit Hilfe von Flockungsmittel wird das Schermetallkonzentrat abgeschieden. Die regenerierten Tonmineralien < 5 µm und das gereinigte Filtrat gelangen in die Vergärung und werden als Dünger verwertet. Das Filtrat wird über einen Ölabscheider geleitet um bei eventuellen Fehlwürfe von Mineralöl eine Belastung bei der späteren landwirtschaftlichen Verwertung des Gärrestes zu vermeiden. Eventuell in den Tonmineralen oder im gereinigten Filtrat vorhandene organische Schadstoffe können durch einen separate biologische anaerobe/aerobe Reinigungsanlage behandelt werden. Diese biologische Behandlung erfolgt ggf. bei Auffälligkeiten von organischen Schadstoffen bei der Qualitätssicherung der Düngerpellets.

Im Allgemeinen werden organische Schadstoffe, die löslich und schwer biologisch abbaubar sind, von Kläranlagen nicht erfasst und sind daher in der EU entweder verboten oder stehen kurz vor einem Verbot. Durch die erfindungsgemäße Verfahrensführung mit durchschnittlich fast einem viertel Jahr Behandlungszeit werden fast alle weiteren, nicht von dem Verbot erfassten, organischen Schadstoffe abgebaut, soweit diese heute bekannt sind. Für Übergangszeiten besteht bei dem erfindungsgemäßen Verfahren die Option, das mechanisch und mit Entwässerungshilfsmitteln gereinigte Abwasser nach einer anaeroben Behandlung gezielt mit spezialisierten, immobilisierten Mikroorganismen zur Reduzierung von organischen Schadstoffen nachzubehandeln.

Die biologische Behandlungszeit von schwer abbaubaren organischen Schadstoffen beträgt in einer kommunalen Kläranlage nur wenige Stunden bei unter 20°C. Im erfindungsgemäßen Verfahren ist die biologische Behandlungszeit mit im Mittel fast ein Vierteljahr bei > 55°C also 200-mal so lang wie in Kläranlagen. Bei Berücksichtigung der RGT-Regel ergibt sich eine um den Faktor 10 größere Abbauleistung.

So können auch heute noch unbekannte organische Schadstoffe besser abgebaut werden. Es entsteht bei dem erfindungsgemäßen Verfahren kein Abwasser. Lediglich ein für die direkte Einleitung gereinigtes Wasser mit hohem Kalium- und Spurenelemente-Gehalt ist für die landwirtschaftliche Verwertung vorgesehen.

### Probleme bei der Hausmüllvergärung

In Deutschland wurden bis heute mechanisch-biologische Restabfallbehandlungskapazitäten für ca. 6 Mio. Tonnen Hausmüll pro Jahr zur Vorbehandlung für die Deponie oder Müllverbrennung installiert. Davon waren 14 Anlagen Hausmüllvergärungsanlagen mit insgesamt 1,5 Mio. Tonnen pro Jahr Durchsatzleistung. 8 Anlagen davon waren Nassvergärungsanlagen mit nassmechanischer Trennung und Perkolation mit einer Durchsatzleistung von insgesamt 850.000 Tonnen pro Jahr.

| **Nassvergärungsanlagen mit nassmechanischer Trennung** | **Jahr IBN** | **Durchsatz t/a** |
|---|---|---|
| MBA Südniedersachsen/Göttingen | 2005 | 133.000 |
| MBA Sachsenhagen | 2005 | 75.000 |
| MBA Wiefels | 2006 | 113.500 |
| MBA Lübeck | 2005 | 140.000 |
| MBA Schwarze Elster | 2004 | 50.000 |

| **Perkolatvergärungsanlagen** | **Jahr IBN** | **Durchsatz t/a** |
|---|---|---|
| ISKA Heilbronn (ISKA) | 2005 | 80.000 |
| ISKA Buchen (ISKA) | 2005 | 160.000 |
| ZAK Kahlenberg (MYT) | 2006 | 100.000 |

| **Trockenvergärungsanlagen** | **Jahr IBN** | **Durchsatz t/a** |
|---|---|---|
| RABA Bassum (Dranco OWS) | 1997 | 112.000 |
| ZAK Kaiserslautern (Dranco OWS) | 1999 | 25.000 |
| MBA Pohlsche Heide (Dranco OWS) | 2005 | 115.000 |
| MBRA-Münster (Dranco OWS) | 2005 | 70.000 |
| aha Hannover (Valorga) | 2005 | 200.000 |
| EVG Rostock (Kompogas) | 2007 | 135.000 |

Diese Hausmüllvergärungsanlagen wurden unter anderem im Förderprojekt 03KB022, gefördert vom deutschen Ministerium für Umwelt, 2013 beurteilt. Die Problematik der Schwimmschichtbildung und Sedimentation in den Gärbehältern trifft auf alle Verfahren zu, wobei Trockenvergärungsanlagen unempfindlicher als Nassvergärungsanlagen sind. Die Trockenvergärungsanlagen Kompogas und Valorga sowie die Nassvergärungsverfahren und Perkolationsverfahren basieren auf einer Entwässerung nach der Vergärung und einer Rückführung der Prozesswässer in die Anmischung vor der Vergärung. Der entwässerte Gärrest enthält immer eine geringere Konzentration der Problemstoffe, wie Feinmineralik, refraktäre organische Belastungen, Stickstoff, Neutralsalze, Schwermetalle, korrosive Bestandteile wie Chlorid, Halogene, Säuren, Sulfat etc., als das Presswasser.

Alle diese Verfahren führen Presswasser zurück (Ausnahme: Dranco). Durch die Rückführung von Presswasser kommt es zur Akkumulation dieser Stoffe, die zu einer starken Hemmung der Biologie der Vergärung führt. Außerdem kommt es zu mechanischen Problemen durch Sedimentation von Feinmineralik und gleichzeitiger Inkrustation durch Ausfällung der steigenden Konzentration von Salzen und gelösten Mineralien. Nassvergärungsverfahren mit Schwerstoffabscheidung benötigen mehr als 3 m³ Presswasser pro Tonne Abfall zur Vergärung. Die Schwerstoffabscheidung erfolgt nur bis zu einer Zielkorngröße von ca. 1,5 mm, es erfolgt somit nur eine Abscheidung von Steinen und Kies. Eine Eindickung der Organik vor der Vergärung erfolgt nicht. Aufgrund des weiteren Viskositätsabbaus durch die Vergärung und - schlimmer noch - durch die nachfolgende Nassoxidation kommt es zu Sedimentation und Schwimmdeckenbildung, die bei einigen Anlagen bis zur Hälfte des ursprünglichen Behältervolumens eingenommen hatten. Hausmüllvergärungsanlagen mit Nassvergärung nach dem Stand der Technik emittieren pro Tonne Abfall im Mittel 700 Liter Abwasser und benötigen Frischwasser zur Verhinderung von Schadstoff- und Störstoffakkumulation in der Vergärung.

Das erfindungsgemäße Verfahren verzichtet demgegenüber vollständig auf eine Rückführung von Prozesswasser nach der Vergärung. Zudem werden die Mineralstoffe Sand (0,1 - 2 mm) und sogar Schluffanteile (10 - 100 µm) abgeschieden und die Organikfraktion wird soweit eingedickt, dass auf eine Entwässerung nach der Vergärung verzichtet werden kann. Wasserüberschuss oder zusätzlicher Wasserbedarf besteht nicht. Die Viskosität in der Vergärung ist bei dem erfindungsgemäßen Verfahren durch die Entwässerung der Feststoffe vor dem Vergärungsverfahren so hoch, dass es durch eventuell eingetragene Schwerstoffe nicht zu Sedimentationsproblemen und durch eingetragene Leichtstoffe nicht zu Schwimmschichtproblemen kommt.

Das erfindungsgemäße Verfahren kommt ohne Gärrestentwässerung und ohne aerobe Gärrestbehandlung aus.

Bei allen bekannten, oben genannten Hausmüllvergärungsverfahren wird eine aerobe Gärrestbehandlung zur Stabilisierung der Feststoffe durchgeführt. Die Abluft aus der aeroben Gärrestbehandlung muss bei allen oben genannten Verfahren, die in Deutschland realisiert wurden, einer Regenerativ-Thermischen Oxidation (RTO) zur Abluftbehandlung zugeführt werden. Der Eigenstrom- und Investitionsbedarf inkl. Gärresteentwässerung, aeroben Gärrestebehandlung Abluftbehandlung und Gärresteabwasserbehandlung beträgt mehr als ein Drittel der Gesamtanlage.

### Vorteile einer heißen und gleichzeitig sauren Abfallwäsche

Hohe Temperaturen begünstigen das nassmechanische Trennverhalten durch eine reduzierte Viskosität des Wassers. Zwischen 20°C und 70°C reduziert sich zum Beispiel die Viskosität von Wasser um 60%.

Hohe Wassertemperaturen begünstigen die Reinigung von Wertstoffen, wie Kunststoffe, Holz und Mineralstoffe, von z.B. Speiseresten, Ölen und Fetten. Der gesamte Abfallmassenstrom wird durch die materialangemessene gesteuerte Haltezeit der Temperatur nach der Wäsche hygienisiert. Problematisch war nach dem Stand der Technik die Verfahrenstechnik der Aufheizung des Abfalls wegen der Inkrustationen bzw. Verkalkungen, die durch die verringerte Löslichkeit von Salzen bei hohen Temperaturen und hohem pH-Wert auftreten. Dieses Problem tritt bei niedrigen pH-Werten trotz hoher Temperatur nicht auf.

Nassvergärungsverfahren nach dem Stand der Technik mischen alkalisches Presswasser aus der Gärresteentwässerung zur Anmischung in die Abfallwäsche zurück, wodurch Inkrustationen im Wärmetauscher bei einer Aufheizung entstehen. Zudem weist das rückgeführte Presswasser aus der Vergärung einen hohen Ammoniumgehalt bei gleichzeitig hohem pH-Wert auf, wodurch erhebliche Ammoniakemissionen in der Abfallwäsche durch die Aufheizung entstehen würden. Viele Vergärungsverfahren mit Rückmischung führen die Wärmeenergie für die Vergärung durch direkte Wasserdampfzugabe zum Substrat oder mittels Beheizung der Gärbehälter zu. Die Inkrustationen können im Wärmetauscher durch Säure und damit Absenkung den pH-Wertes verhindert werden. Dies schließt jedoch wiederum die Nutzung von Presswasser aus der Gärresteentwässerung aus verschiedenen Gründen aus:
- Aufgrund des hohen pH-Wert der Gärreste von 8 bis 8,5 mit starker Pufferwirkung ist eine sehr große Säuremenge zur pH-Wert Senkung auf < 5 erforderlich. Der frische Abfall weist im Vergleich zum Gärrest einen niedrigen pH-Wert < 6, teilweise < 5 auf und hat eine geringe Pufferwirkung.
- In der Vergärung wird die Biomasse zersetzt und Schadgase wie H₂S, Ammoniak und Methan sowie geruchsintensive Stoffe wie Mercaptane werden gebildet. Diese Schadgase werden teilweise mit dem Biogas diffus bei der nassmechanischen Trennung, Entwässerung oder Gärresthandling ausgetragen und gelangen ungereinigt in die Umgebung. Gärreste werden vor der landwirtschaftlichen Verwertung abgekühlt und der pH-Wert nicht verändert. Werden Gärreste jedoch erhitzt und der pH-Wert auf < 5 reduziert, würde dies wegen der freiwerdenden Schwefelgase zu einer erheblichen Geruchsentwicklung in der Abfallwäsche und allen nachfolgenden Anlagenteilen führen. Zudem können durch Methan und methanbildende Mikroorganismen explosible Gasgemische entstehen.

Bei Temperaturen von größer 70°C, wie bei dem erfindungsgemäßen Verfahren, können biologische Aktivitäten wie Hydrolyse und Biogasproduktion sicher ausgeschlossen werden. Zudem ergibt sich bei einer Verweilzeit von mehr als einer Stunde für Materialien < 12 mm eine gleichzeitige Hygienisierung. Bei pH-Werten < 5 können biologische Abbaureaktionen, die Methan und Geruchsstoffe bilden, ebenfalls stark reduziert werden. Dieser Effekt wird zum Beispiel bei der Grassilierung oder beim sauer Einlegen genutzt.

### Aufgabenstellung der Erfindung:

Siedlungsabfälle sowie gewerbliche Abfälle sollen - nachdem alle Möglichkeiten der Vermeidung und Wiederverwendung ausgeschöpft wurden - recycelt werden. Das "Recycling" (stoffliche Verwertung) steht dabei in der Abfallhierarchie der EU vor "Recovery" ( energetische oder sonstige Verwertung). Eine direkte Deponierung oder direkte Verbrennung von Abfall kann nicht mehr die Anforderungen der Kreislaufwirtschaft der Europäischen Union erfüllen.

Folgende Ziele einer zukunftsfähigen Abfallwirtschaft können mit dem erfindungsgemäßen Verfahren erreicht werden:

### Urban Mining

Durch die Abfallaufbereitung sollen die Wertstoffe aus dem Siedlungsabfall lokal verwertet werden und so die natürlichen Ressourcen schonen. Edelmetalle wie Aluminium dürfen nicht durch thermische Verfahren verdampft werden und in Filterstäube gelangen, die dann als Sonderabfall entsorgt werden. Allein der kumulierte Energieaufwand KEA nach VDI 4600, Januar 2012, bei der Rückgewinnung von 0,7 Gewichtsprozent Nichteisenmetallen (KEA Aluminium beträgt 200.000 kJ/kg) und 3,5 Gewichtsprozent Eisen (KEA von Eisen beträgt 36.000 kJ/kg) aus den gemischten Siedlungsabfällen beträgt ca. 25% des unteren Heizwertes von ca. 9.000 - 10.000 kJ/kg) der gemischten Siedlungsabfälle. Das Potential für das Kunststoffrecycling beträgt bei 15 Gewichtsprozent Kunststoffen (KEA ca. 100.000 kJ/kg) im gemischten Siedlungsabfall nach der Berechnung des KEA ca. 50% des unteren Heizwertes der gemischten Siedlungsabfälle. Der KEA zur Kunststoffproduktion in Deutschland, Stand 2009, entspricht dem eineinhalbfachen der Stromproduktion aus deutschen Kernkraftwerken. Gewinnt man zusätzlich das Biogaspotential mit 10% des Abfallgewichtes zurück, sind dies weitere 35% des unteren Heizwertes der gemischten Siedlungsabfälle. Schon wenn nur 30% des Siedlungsabfalls recycelt werden, können 110% des KEA von gemischtem Siedlungsabfall zurückgewonnen werden. Neben der Notwendigkeit des Recyclings von Wertstoffen - aufgrund des hohen Energieaufwands bei deren Produktion - besteht darüber hinaus die Notwendigkeit des Recyclings von Ressourcen aufgrund des endlichen Vorkommens dieser Ressourcen auf unserem Planeten. Hierzu gehörten bis 2014 im Wesentlichen Antimon, Beryllium, Flussspat, Gallium, Germanium, Graphit, Indium, Kobalt, Magnesium, Niob, Metalle der Platingruppe, schwere seltene Erden, leichte seltene Erden und Wolfram. Erst 2014 sind Borate, Chrom, Kokskohle, Magnesit, Phosphatgestein und metallisches Silizium in die Liste kritischer Elemente der Europäischen Kommission (European Commission MEMO/14/377) neu aufgenommen worden. Durch das erfindungsgemäße Verfahren können die kritischen Elemente aus Siedlungsabfällen zu fast 100% zurückgewonnen werden. So wird zum Beispiel Phosphat in der Düngemittelfraktion, Silizium als Sand, Borate in der Holzfraktion und in der Keramik- und Steingutfraktion, Antimon in den Kunststofffraktionen usw. zurückgewonnen. Gelöste kritische Elemente werden im Waschwasser aufkonzentriert und können als Konzentrat in speziellen metallurgischen Anlagen aufbereitet werden. Neben den Schwermetallen können auch die kritischen Elemente so zurückgewonnen werden.

### Zero Waste

Ziel der Weiterentwicklung in der Abfallwirtschaft ist die komplette Schließung der Stoffkreisläufe und damit eine 100%ige Abfallverwertung. So wird nicht nur die Rückgewinnung von Phosphaten aus dem Klärschlamm und Abwasser der Kläranlagen - ca. 85% des Phosphates aus den Haushalten - gefordert, sondern auch die 15% des Phosphates aus dem festen Hausmüll soll als organischer Dünger zurückgewonnen werden. In Deutschland hat man 30 Jahre gebraucht, um 50% der Haushalte an die separate Bioabfallsammlung anzuschließen. Dennoch können maximal - auch bei 100% Anschlussquote der Haushalte an die Bioabfallsammlung - nur 50% der Phosphate über die Bioabfallbehandlung für die Landwirtschaft zurückgewonnen werden. Der Rest wird über Verpackungsabfälle und den Restabfall über z.B. Müllverbrennungsanlagen entsorgt und geht dem Nahrungsmittelkreislauf endgültig verloren. Nur durch ein vollständiges Recycling der organischen Abfälle kann der weitere Verlust des kritischen Elementes Phosphor vermieden werden. Aktuell fordert die Europäische Union dies im Vorschlag für eine Richtlinie des Europäischen Parlaments und des Rates zur Änderung der Richtlinie 2008/98/EG über Abfälle "COM(2015) 595 final" vom 02.12.2015 und in er Mitteilung der Europäischen Kommission vom 02.12.2015 "Den Kreislauf schließen - Ein Aktionsplan der EU für die Kreislaufwirtschaft" "COM/2015/0614 final". Auch neben dem 100%igen Recycling der kritischen Elemente sollten alle nicht vermeidbaren und nicht wiederverwendbaren Stoffströme des Siedlungsabfalls soweit aufbereitet und von Schadstoffen befreit werden, dass man am Verwertungsort einen Erlös erzielt. Nur so kann man von einem Wert des Abfalls sprechen.

### Zero Emission

CO₂-Emissionen aus fossilen Brennstoffen sollten weitestgehend vermieden werden. Dies ergibt sich aus dem Nationalen Beitrag der EU (INDC) zur Klimarahmenkonvention (UNFCCC) am 06.03.2015 zur Umsetzung des Zieles von 40% Reduzierung der Treibhausgasemissionen bis 2030 im Vergleich zu 1990. Im Dezember 2015 wurde auf der Klimakonferenz in Paris beschlossen, dass die Erwärmung der Welt auf weniger als 2 °C begrenzt werden soll. Die globalen Netto-Treibhausgasemissionen sollen hierzu in der zweiten Hälfte des 21. Jahrhunderts auf null reduziert werden. Finanzhilfen für die Entwicklungsländer wurden ebenfalls vereinbart. Die Verbrennung von regenerativen Brennstoffen wie Biogas aus der Vergärung von Abfällen für die Strom- und Wärmeversorgung der Anlage ist gewünscht, soweit der Wärmebedarf nicht durch Sonnenkollektoren in Kombination mit Wärmespeichern und soweit der Strombedarf nicht über Photovoltaik und Windenergie lokal erzeugt werden kann. Wo möglich, sind Elektromotoren auch für mobile Geräte zu bevorzugen, die ihren Strom aus regenerativen Energiequellen beziehen. Radlader oder andere mobile Geräte, die mit Diesel betrieben werden müssten, sind zu vermeiden. Der Biogasüberschuss, der nicht für die Strom- und Wärmeversorgung benötigt wird, sollte für den Betrieb der Sammelfahrzeuge und des öffentlichen Nahverkehrs eingesetzt werden. Nach dem Aktionsplan der EU für die Kreislaufwirtschaft" "COM/2015/0614 final" der Europäischen Kommission vom 02.12.2015 sind auch die Produkte so herzustellen, dass diese recycelt werden können. Soweit Produkte am Ende ihres Lebenszyklus nicht mit zumutbarem Aufwand der Wiederverwendung zugeführt werden können, dürfen diese Produkte nicht mehr vertrieben werden. Eine Verbrennung technisch nicht stofflich verwertbarer Abfälle ohne eine Umstellung der Produkte durchzuführen, ist nicht mehr zielführend.

### Zero Waste Water

In der Mitteilung der Europäischen Kommission vom 02.12.2015 "Den Kreislauf schließen - Ein Aktionsplan der EU für die Kreislaufwirtschaft" "COM/2015/0614 final" wird die Wiederverwendung von Abwasser in der Landwirtschaft gefordert. "*Die Wiederverwendung von Wasser in der Landwirtschaft trägt auch zum Nährstoffrecycling bei (Ersetzung von festen Düngemitteln). Die Kommission wird eine Reihe von Maßnahmen zur Förderung der Wiederverwendung von aufbereitetem Abwasser treffen und u.a. Rechtsvorschriften über Mindestanforderungen für wiederverwendetes Wasser ausarbeiten*." Eine aktuelle Studie (Engeli H., Baier U., Edelmann W., Rüsch F., Strebel S. (2013): Nachbereitung von Gärgut, Schlussbericht, Bundesamt für Energie, Bern, Schweiz) zeigt, dass der Aufwand der Reinigung von Abwasser aus Vergärungsanlagen zur Reduzierung des Transportaufwandes für Düngemittel so hoch ist, dass dies erst ab Transportentfernungen von über 200 km ökologisch und ökonomisch zu rechtfertigen ist.

### Das erfindungsgemäße Verfahren harmoniert mit getrennter Sammlung und Abfallvermeidung

### Separate Sammlung

Trotz der 100%igen Recyclingleistung des erfindungsgemäßen Verfahrens, auch für nicht separat gesammelten Mischabfall, ist aus abfallpolitischen Gründen zur Einhaltung der Abfallhierarchie eine separate Abfallsammlung folgender Abfälle in der Europäischen Union unerlässlich:
1. Pappe und Papier - ein Papierrecycling ist nur bei separater Erfassung möglich. Die Vergärung von Papier stellt nur eine Kaskadennutzung mit energetischer Verwertung (Biogas) und Düngung dar.
2. Elektro- und Elektronikaltgeräte sowie Sperrabfall - Vorbereitung zur Wiederverwendung ist nur bei separater Erfassung möglich
3. Verpackungsabfälle -Die Kaufentscheidung wird z.B. durch Verpackungssteuer, die eine Recyclinggebühr beinhaltet, beeinflusst, um Verpackungsabfälle zu vermeiden. Verpackungsabfälle werden somit ohne weitere Entsorgungsgebühr durch separate Erfassung entsorgt.
4. Bio- und Gartenabfälle - Garten und Bioabfälle sind mit Ausnahme von überlagerten Lebensmitten nicht vermeidbare Abfälle, die nur aufgrund von Eigenkompostierung statistisch nicht vollständig erfasst werden. Unsachgemäße Eigenkompostierung zum Zweck der Abfallvermeidung stellt eine vermeidbare Emission in Luft, Boden und Grundwasser, ähnlich den Emissionen einer Deponie, dar. Es sollten daher keine oder nur sehr viel geringere Entsorgungsgebühren als für Restabfall erhoben werden
5. Restabfall - die Gebühren für Restabfall sollten so gewählt werden, dass ein Gleichgewicht zwischen der Menge und der Qualität der separat gesammelten Abfälle entsteht und illegale Entsorgung von Hausmüll in der Umwelt verhindert wird

Restabfall, Verpackungsabfälle, Bioabfall und Sperrabfall können von dem erfindungsgemäßen Verfahren flexibel als Gemisch oder auch separat zu verschiedenen Betriebszeiten mit der identischen Anlage verwertet werden.

Bei Veränderung der separat gesammelten Abfallmengen und -qualitäten werden nur die Betriebszeiten und auch die Output-Qualitäten verschoben, es kommt aber nicht zu einer Über- oder Unterauslastung durch veränderte Erfassungsquoten.

Mit fortschreitender Abfallvermeidung können die freien Kapazitäten der Anlage z.B. für den Rückbau der vorhandenen Deponien zum Recycling des Deponats genutzt werden.

Allen Abfällen ist gemeinsam, dass verwertbare Fraktionen nur mit einer nassmechanischen Trenntechnik erzeugt werden können, wie folgende Tabelle aufzeigt:

| **Abfallart** | **Empfehlung nassmechanisches Trennverfahren als bestes Behandlungsverfahren** |
|---|---|
| Restabfall | Bundesamt für Umwelt Schweiz (2009), Stadt Moskau (2009), Kläranlageverband Schaffhausen (2008), Universität Ljubljana (2010), Umweltbundesamt Deutschland (2001, 2013), Gewitra (2013), Waste Consult (2013, 2015) |
| Bioabfall | Umweltbundesamt (2015), ia GmbH (2015), Prognos AG (2015) |
| Verpackungsabfälle | Vorwaschen, Heißwaschen und Schwimm-Sink-Trennung sind aktuelle Voraussetzungen für das Kunststoffrecycling. Seit 2014 (Green-fence-policy China) ist kein Absatz für schmutzige Kunststoffabfälle mehr möglich! |
| Deponat | Leitfaden Landfill Mining mit nassmechanischem Trennverfahren, Hrsg.: Umweltministerium NRW (2016), RWTH Aachen (2012), IfEU Institut für Energie und Umwelt Heidelberg GmbH (2013), TU Braunschweig (2015), RuK (2016), Ökoinstitut e.V. (2016), TU Clausthal (2016) |

### Vorteile des erfindungsgemäßen Verfahrens

Auf < 80 mm zerkleinerte Abfälle werden in einem turbulenten Mischer mit sehr viel heißem Wasser gemischt, auf 75 °C temperiert und durch Zugabe von selbst produzierter Schwefelsäure wird ein pH-Wert von 5, dies entspricht dem pH-Wert von Bier, eingestellt.

Alle übelriechenden Geruchstoffe und Lösemittel werden in dieser Stufe verdampft und über einen Wäscher gereinigt. Stickstoff verbleibt trotz der hohen Temperaturen durch die Säurezugabe im Abfall.

In der ersten Stufe wird in einer Abscheidekammer eine Strömung so eingestellt, dass ein Stein mit 2 cm Durchmesser zu Boden sinkt, eine Kartoffel und selbst

Hartkunststoffe wie PVC jedoch in den Überlauf gespült und bei ca. 30 mm abgesiebt werden. Die organischen Leichtstoffe, bestehend aus Kunststoffen, Biomasse und Holz, gelangen in einen Dosierbunker.

In einer zweiten Abscheidekammer wird die Strömung so eingestellt, dass ein Kieskorn mit 2 mm Durchmesser zu Boden sinkt, ein Kirschkern oder Pflaumenkern oder Hartkunststoff mit 2 cm Durchmesser in den Überlauf gelangt. Der Überlauf wird bei ca. 8 mm gesiebt und ebenfalls in den Dosierbunker gefördert.

Die Suspension < 8 mm wird über einen Zyklon von Sand und Feinsand befreit und wieder vorne zur Anmischung verwendet.

Es wird kein zusätzliches Wasser benötigt. Der Sand und der Feinsand werden über eine Goldwaschtechnik bis zur Verwertungsqualität für die Bauindustrie aufbereitet. Die Schwermetalle werden durch weitere Zugabe von Säure gelöst und als verwertbares Schwermetallkonzentrat abgeschieden. Eventuelle organische Schadstoffe, die nicht durch die Vergärung zerstört werden können, können alternativ mit Verfahren der biologischen Bodenreinigung entfernt werden. Schadstoffe wie z.B. Dioxine und DDT, die auch mit dem erfindungsgemäßen Verfahren nicht entfernt werden können, kommen im Siedlungsabfall schon seit vielen Jahren nicht mehr vor. Nur bei älteren Abfällen, wie aus dem Deponierückbau, sind unter Umständen weitergehende biologische Sanierungsmaßnahmen - analog zu Altlasten - erforderlich. Aus den Mineralstoffen werden sämtliche Metalle zurückgewonnen. Durch moderne optische Sortiersysteme kann auch Glas nach Farben getrennt und wiederverwertet werden. Kies und Steine können als Recyclingbaustoffe eingesetzt werden.

Die mineralstoff-freie Überlauffraktion, die in den Dosierbunkern hygienisiert und eingeweicht wurde, wird mit dem patentierten Zelllyse-Verfahren nachbehandelt:

### Zelllyse-Verfahren

Pflanzliche Zellen haben bei Temperaturen von 70 °C weiche Zellwände, die durch einfache Scherwirkung einer Schneckenpresse gebrochen werden können. Die vergärbaren Bruchstücke haben nur noch eine Faserlänge von 2-3 mm und können durch Siebung bei 8 mm von recyclingfähigen Kunststoffen, Holz und Textilien abgetrennt werden. Auch das eingeweichte Papier und die Inhalte von Windeln ohne die Kunststofffolien gelangen in die Vergärung. Die in den Windeln befindlichen Superabsorber, die viel Flüssigkeit aufnehmen können, sind völlig ungiftig und werden heute zu hohen Preisen als Geo-Humus zur Bodenverbesserung verkauft. Das nicht separat gesammelte Papier wird als Biogas und Dünger verwertet.

### Kunststoffaufbereitung

Durch computergesteuerte, automatische Sortiersysteme werden sortenreine Kunststoffgranulate erzeugt. Je nach Standort können direkt vermarktbare Kunststoffprodukte oder Kunststoffgranulate produziert werden. Holzanteile werden für die Spanplatten- oder MDF-Produktion aussortiert. Das restliche Holz wird zusammen mit den Textilien für die Herstellung von faserverstärkten Kunststoffen eingesetzt. Diese sind unempfindlich gegen Feuchtigkeit und sehr robust, daher steigt die Nachfrage kontinuierlich, z. B. beim Ersatz von Eichenholz im Wasserbau, Terrassen, Fahrradwegen etc.. Die Qualität dieser faserverstärkten Kunststoffe wird durch garantierte Festigkeitsstandards gesichert.

### Vergärung

Die störstofffreie Abfallsilage < 8 mm wird in Dosierbunkern wie Maissilage gelagert. Die thermophile Trockenvergärung mit Pfropfen-Strömung hat eine zusätzliche Hygienisierungswirkung. Es können hier auch landwirtschaftliche schadstoffarme Reststoffe wie Gülle oder schadstoffarmer Belebtschlamm aus der Abwasserreinigung verwertet werden.

### Biogasnutzung und Energieerzeugung

Schwefelwasserstoff wird aus dem Biogas als verdünnte Schwefelsäure abgeschieden und zu Schwefelsäure aufkonzentriert. Die Schwefelsäure wird zur Schwermetallauswaschung und für die Düngerproduktion eingesetzt. Das gereinigte Biogas kann zur Kraft-Wärme-Kopplung vor Ort eingesetzt werden oder als Biomethan vermarktet werden. Alle Antriebe der Abfallbehandlungsanlage sollten elektrisch ausgeführt werden. Mobile Geräte sowie die Sammelfahrzeuge werden entweder elektrisch oder mit Biogas betrieben.

Alle Dachflächen der Anlage werden für Photovoltaik und Solarthermieanlagen in Kombination mit Wärmespeichern für den Eigenbedarf an Strom und Wärme genutzt.

### Düngerproduktion

Nach der Entwässerung des Gärrestes wird die Flüssigkeit durch Laugenzugabe (NaOH) und Filterpresse vom Phosphat und durch Strippung vom Stickstoff befreit. Stickstoff wird zusammen mit der Schwefelsäure als Ammoniumsulfatdünger vertrieben. Die Feststoffe werden getrocknet, pelletiert und, je nach Anforderungen mit weiteren Düngern gemischt, vermarktet. Kalium und andere wichtige Spurenelemente werden mit der schadstofffreien geklärten Flüssigkeit auf naheliegenden Flächen durch Beregnung verwertet.

### Schwermetalle

### Problematik in herkömmlichen Abfallbehandlungsanlagen:

Lösliche Schwermetalle liegen als positiv geladene Ionen vor und lagern sich entweder an negativ geladene Tonminerale oder an negativ geladene Bioschlämme, die sich bei biologischer Behandlung bilden. Diese Schleime ((Extrazelluläre polymere Substanzen) schützen die Bakteriengesellschaften vor Temperatur- und Feuchtigkeitsschwankungen, vor Säuren und Laugen. Schwermetalle werden zum Schutz der Zellen in der Schleimhülle fixiert. Diese Substanzen nach der biologischen Behandlung können deshalb weder mit Säurezugabe noch durch Kochen von Schwermetallen befreit werden. Daher können Gärreste aus konventionellen Bioabfall- und Restabfallvergärungsverfahren und Klärschlämme nicht von Schwermetallen befreit werden.

### Fortschritt durch das erfindungsgemäße Verfahren:

Ohne biologische Behandlung sind die Schwermetalle zum größten Teil an Tonmineralien fixiert. Tonminerale wirken als Ionenaustauscher, die mit Säure regeneriert werden können. Dabei werden die Schwermetalle wieder frei und in der Hüttenindustrie recycelt.

### Feststoffe aus kommunalem Abwasser

Klärschlämme bestehen zu 50% aus Feinsand und Tonmineralen und zu 50% aus Bioschlamm. Eine landwirtschaftliche Verwertung von Klärschlamm wird durch den hohen Schwermetallgehalt des Klärschlammes erschwert. Nur eine Abscheidung aller Feststoffe, inkl. der schwermetallhaltigen Tonminerale, aus dem Abwasser vor der biologischen Behandlung ermöglicht eine Schwermetallabscheidung.

Eine Schadstoffentfrachtung von kommunalem Abwasser durch das erfindungsgemäße Verfahren würde die Kosten für Sanierung, Neubau und Betrieb kommunaler Kanalsysteme senken. Die zentralisierte Abwasserbehandlung in großflächigen Kläranlagen außerhalb der Stadt kann durch kompakte Kläranlagen mit zentraler Feststoffverwertung, gemeinsam mit der Abfallbehandlung, ersetzt werden. Beispielsweise kann ein marodes Mischwasserkanalsystem für Regenwasser und das in dezentralen Anlagen gereinigte Schmutzwasser ohne Sanierung weiter genutzt werden. Die Undichtigkeit des alten Kanalsystems fördert zudem eine gewünschte Versickerung. Für das kommunale Schmutzwasser sind nur noch sehr viel kleinere und damit kostengünstigere Kanaldimensionen und kleine dezentrale Anlagen erforderlich.

Haupterfolg dieser Maßnahmen ist eine 100%ige Klärschlammverwertung ohne Klärschlammverbrennung, ohne Deponie und ohne Erzeugung von Sondermüll.

### Hygienisierung

Eine Verbreitung von Antibiotika-Resistenzen entsteht durch bereits resistente Darmbakterien. Diese Bakterien werden in der konventionellen Kläranlage nur in geringem Maße abgetötet und gelangen in den Ablauf der Kläranlage und in den Klärschlamm. Durch Gen-Austausch mit anderen Bakterien kommt es zu einer unkontrollierten Verteilung des Resistenz-Genes in der Umwelt.

Das erfindungsgemäße Verfahren hygienisiert alle Fraktionen schon vor der Vergärung und zusätzlich während der Vergärung. Durch die Feinfiltration mit Flockungshilfsmittel in Kompakt- Kläranlagen kann der größte Teil der Mikroorganismen abfiltriert werden. Das Filtrat kann weiter gereinigt und durch UV-Bestrahlung hygienisiert und lokal genutzt werden.

### Deponat aus Deponierückbau

Grundsätzlich unterscheidet sich die Zusammensetzung einer Deponie nicht vom damals deponierten Material. Lediglich leicht abbaubare Biomasse wurde in 100-mal längerer Zeit als in einer Abfallbehandlungsanlage abgebaut und in Biogas umgewandelt. Zusätzlich wurde bei der Deponierung Bodenmaterial für die Befahrung und als Zwischenabdeckung mit eingebaut. Die recyclingfähigen Stoffe wie Sand, Kies, Steine, Glas, Metalle, Kunststoffe, Holz und Textilien haben sich kaum verändert. Bioschlamm hat sich nur im Verhältnis ca. 1/50 bis 1/100 im Vergleich zu einer technischen Vergärung gebildet. Gelöste Schwermetalle sind über das Sickerwasser an die Tonmineralien des Abfalls und der Böden zur Zwischenabdeckung gebunden und können mit dem erfindungsgemäßen Verfahren herausgelöst und verwertet werden.

### Beschreibung des Verfahrens

Das erfindungsgemässe Verfahren wird in den angehängten Ansprüchen definiert.

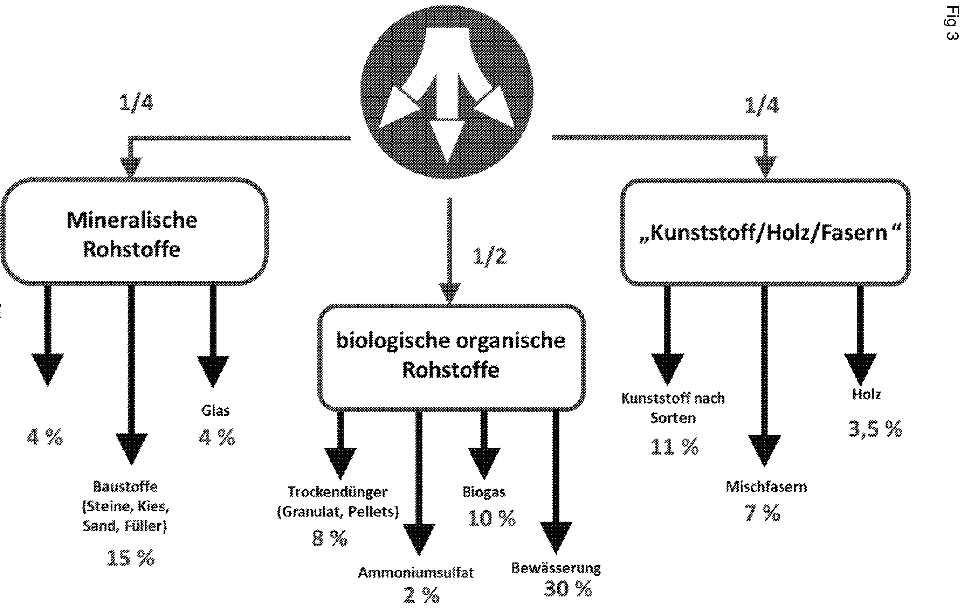

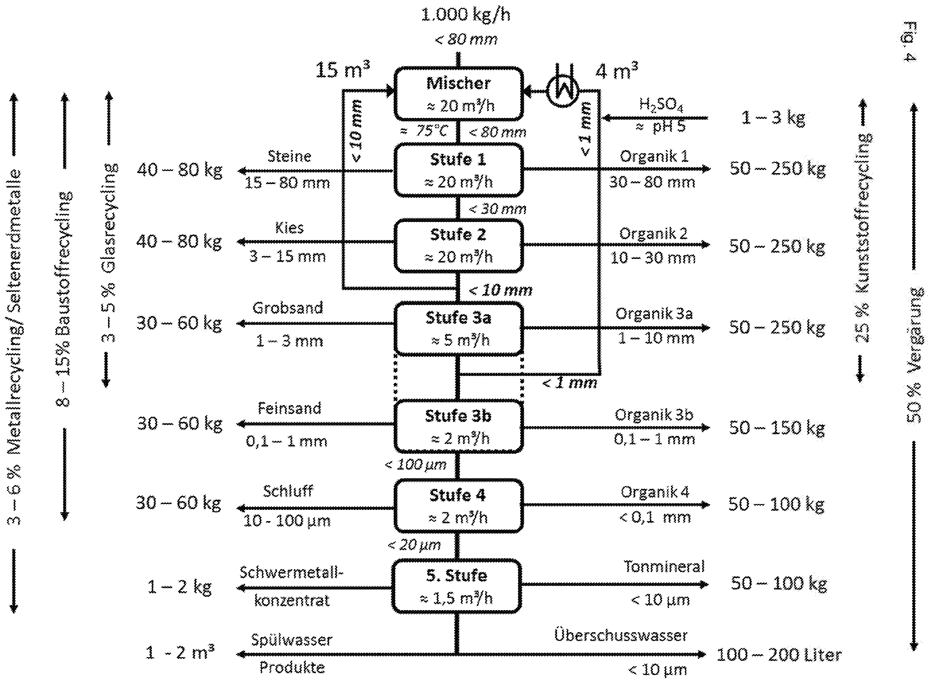

Das Verfahren wird durch die Zeichnungen Fig.1 bis Fig 4 sowie die Legende zu Fig. 1 und Fig. 2 dargestellt.
Fig 1: Überblick Gesamtverfahren
Fig.2: Detail Schwermetallabscheidung
Fig. 3: Idealisierte Massen und Produktströme
Fig. 4: Idealisierte Massen und Produktströme der nassmechanischen Trennung

### Trockenaufbereitung

Die Annahme und Lagerung des Abfalls erfolgt in einem Tiefbunker. Über ein halbautomatisches Greifersystem (1) wird der Zerkleinerer (2), ein Langsamläufer mit einer brechend-quetschenden Zerkleinerungswirkung, beschickt. Der zerkleinerte Abfall wird auf 60 - 100 mm, vorzugsweise auf 80 mm mit einem Trommelsieb (3), Vibrations- oder Sternsieb gesiebt. Der Siebüberlauf größer 60 - 100 mm wird optional mittels Überbandmagneten von Eisenmetallen befreit, um Verschleiß bei der weiteren Zerkleinerung (4) zu vermeiden. Optional kann der Siebüberlauf zur weiteren automatischen Sortierung auf 250 - 350 mm gesiebt werden und aus der Fraktion größer 60 bis 100 mm und kleiner 250 - 350 mm können über automatische Sortiersysteme Wertstoffe wie Tetrapack, PET-Flaschen, Holz etc. aussortiert werden. Der Siebüberlauf wird mit einem schneidenden Zerkleinerer (4) mit Siebkorb auf kleiner 60 - 100 mm, vorzugsweise 80 mm zerkleinert.

Alle Fraktionen kleiner 60 bis 100 mm, vorzugsweise 80 mm (5) gelangen in die nassmechanische Trennung.

### 1. Stufe nassmechanische Trennung (11)

Der Abfall wird in einem Mischer (6) mit obenliegenden Rührern mit heißem Kreislaufwasser (7) und (25.1) gemischt. Das Mischungsverhältnis beträgt 1:10 bis 1:30, vorzugsweise 1:20. Die Temperatur der Mischung beträgt ca. 75°C und wird über den Wärmetauscher (9) geregelt. Zur Auswaschung von Schwermetallen aus den Feststofffraktionen wird durch Zugabe von Schwefelsäure (10), der pH-Wert der Suspension auf 5 bis 6, vorzugsweise ein pH-Wert 5, eingestellt. Vorzugsweise soll die Schwefelsäure, die bei der Biogasentschwefelung produziert wird, verwendet werden. Bei der Temperaturerhöhung auf ca. 75°C kann die Geruchsbelastung durch Ammoniak nur durch die pH-Wert-Absenkung vermieden werden. Ebenfalls wird die Verkalkung des Wärmetauschers durch einen niedrigen pH-Wert vermieden.

Zur Mischung mit dem Mischer (6) werden mehrere schnelllaufende Schneckenförderer (MTS-Mamut-Turbo-Schneckenpumpe) mit ca. 1 - 1,5 m Flügelsteigung und einer Drehzahl von 150 bis 300 Umdrehungen pro Minute eingesetzt. Die MTS-Rührer sind fliegend gelagert und mit einem Frequenzumrichter ausgestattet. Die MTS-Rührer haben zwei oder mehr Flügel und werden von oben angetrieben. Diese Art der Mischung sorgt für eine intensive Auflösung der Leichtstoffe ohne zu zerkleinern und verhindert eine Zopfbildung. Durch die obenliegenden Antriebe und die Vermeidung der Mischung von Schwerstoffen haben diese Rührer einen sehr geringen Verschleiß.

Die Mischung wird in die Abscheidekammer 1 (12) überführt, die ebenfalls mit einem MTS-Rührer ausgestattet ist. Durch eine einstellbare Strömung durch den MTS-Rührer werden Schwerstoffe Inert 1 (14) bis 15 mm Korngröße in der Abscheidekammer 1 (12) abgeschieden. Die Strömung wird so eingestellt, dass ein Stein größer 15 mm abgeschieden wird, jedoch sich z.B. eine Kartoffel oder Hartkunststoffe nicht absetzen und mit der Suspension ausgetragen werden. Die Schwerstoffe Inert 1 (14) werden über einen, mit mechanisch gereinigtem Kreislaufwasser gegengespülten, Schneckenförderer ausgetragen. Die Geometrie des Schneckenförderers und die Gegenspülung sind so bemessen, dass eine weitere Reinigung der Schwerstoffe mit Kaskadenspülung mit gereinigtem Kreislaufwasser erfolgt.

Die verbleibende Suspension wird durch ein Sternsieb oder Spannwellensieb (13) bei 25 bis 35 mm gesiebt. Die Organikfraktion größer 25 bis 35 mm Organik 1 (26) wird direkt oder nach einer einfachen Entwässerung in die Niedertemperaturgarung (64) gefördert.

### 2. Stufe nassmechanische Trennung (22)

Die Suspension (21) kleiner 25 - 35 mm wird in die Abscheidekammer 2 (23) überführt. In der Abscheidekammer 2 werden Schwerstoffe Inert 2 (15) größer 2 bis 4 mm abgeschieden. Die Strömung ist so einzustellen, dass Kies bis 2 bis 4 mm abgeschieden wird, jedoch z.B. ein Kirschkern oder ein Hartkunststoff mit der Suspension ausgetragen wird.

Die Suspension wird dann bei 4 bis 12 mm, vorzugsweise 8 mm, mittels eines Spannwellensiebes (24) gesiebt. Die Leichtstoffe Organik 2 (27) werden direkt oder nach einer einfachen Entwässerung in die Niedertemperaturgarung (64) gefördert.

### 3. Stufe nassmechanische Trennung (28)

Die Suspension kleiner 4 bis 12 mm (25) wird in eine Pumpenvorlage überführt. In der Pumpenvorlage werden Schwerstoffe zum Verschleißschutz der nachfolgenden Pumpe abgeschieden. Die Strömung ist über die MTS-Rührer so einzustellen, dass Sand bis 1 mm abgeschieden wird, jedoch ein Zitronenkern oder Hartkunststoff mit der Suspension ausgetragen wird. Die Schwerstoffe gelangen direkt in die weitere Waschung in die Abscheidekammer 3 (35.1). Die Strömung und Geometrie der Pumpenvorlage wird so eingestellt, dass ausschließlich sehr leichte Kunststoffe wie Schaumstoff, Styropor oder geschäumtes Polyurethan als Schwimmschicht ausgetragen werden können. Die Schwimmschicht wird nach einer mechanischen Entwässerung in die Kunststoffaufbereitung überführt.

Die Suspension wird über eine Pumpe einem Hydrozyklon (29) zugeführt.

Durch den Hydrozyklon (29) werden die Mineralstoffe > 1 mm im Unterlauf (29.1) konzentriert. Leichte Mineralik im Überlauf (29.2), wie z.B. Blähton oder Eierschalen, die nicht sicher bei 1 mm abgeschieden werden, stören in der Vergärung nicht und wirken sich positiv auf den Dünger aus. Gleichzeitig wird die Feinmineralik im Unterlauf von 7% bis 15%, vorzugsweise 10% der Kreislaufwassermenge konzentriert. Der Hydrozyklon ermöglicht durch Anpassung von Überlauf und Unterlauf einen Einfluss auf die Unterlaufmenge. Neben der Feinmineralik < 1 mm wird auch ein Anteil der Feinorganik < 1 mm im Hydrozyklonunterlauf (29.1) ausgetragen. Der Hydrozyklonunterlauf wird komplett aufbereitet und auch die Feinorganik abgeschieden, wie im Folgenden beschrieben. So wird einer Akkumulation von Feinmineralik im Kreislaufwasser entgegenwirkt, obwohl nur für ca. 10% des Kreislaufwassers die Feinorganik < 1 mm abgeschieden wird. Diese Verfahrensweise reduziert die Investitions- und Betriebskosten erheblich.

Der Hydrozyklonüberlauf (29.2) wird bei 1 mm bis 2 mm, vorzugsweise 1,5 mm, mittels eines Spannwellensiebes (30) gesiebt.

Mit einer Pumpe wird die Suspension (33) < 1,5 mm über einen Plattenwärmetauscher (9) auf ca. 85°C erhitzt und als Kreislaufwasser (7) zur Abfallmischung (6) und Spülung zurückgeführt.

Die Organik 3a (30.1) wird der Schneckenpresse Organik 3 (32) zugeführt, entwässert und das Pressgut (34) in die Feststoffvorlage Vergärung (72) befördert.

Der Zyklonunterlauf (29.1) wird ebenfalls bei 1 mm gesiebt und in einem Sandwäscher (35.1) gereinigt.

Die Suspension (35.2) < 1 mm wird über Wendelscheider 1 (36) gereinigt. Die Leichtfraktion (36.2) aus dem Wendelscheider (36) wird bei 100 µm gesiebt und die Organik 3b (31.1) wird der Schneckenpresse Organik 3 (32) zugeführt. Die Mittelfraktion wird zurückgeführt zum Wendelscheider 1 (36). Die Schwerfraktion (36.1) wird bei 100 µm gesiebt und der Sand wird gemeinsam mit der Schwerfraktion aus der Abscheidekammer 3 (35.1) nachgewaschen.

### 4. Stufe nassmechanische Trennung (41)

Die Suspension (39) < 100 µm aus der Schwerfraktion wird mittels Ultraschall (47) behandelt und dem Wendelscheider 2b (48) zugeführt.

Die organische (36.2) und die mineralische (36.1) Fraktion aus dem Wendelscheider (36) werden jeweils separat auf < 50 µm bis < 300 µm, vorzugsweise < 100 µm, gesiebt (Siebe 31 und 37) und der Siebdurchlauf (39 und 40) nochmals jeweils separat mit einem Wendelscheider (42 und 48) gereinigt, wobei jeweils eine überwiegend mineralische Fraktion (42.1 und 48.1) sowie jeweils eine überwiegend organische Fraktion (42.2 und 48.2) entstehen. Der mineralische Anteil aus dem Wendelscheider (42) der überwiegend organischen Fraktion (40) wird dem Wendelscheider (48) der mineralischen Fraktion (39) gemeinsam mit der überwiegend mineralischen Fraktion (42.1) zugegeben und dies wird kreuzweise auch mit der organischen Fraktion (48.2) aus dem, mit der überwiegend mineralischen Fraktion betriebenen, Wendelscheider (48) durchgeführt.

Die mineralische Fraktion (48.1) kleiner 50 µm bis 150 µm, vorzugsweise kleiner 100 µm wird mittels Vakuumbandfilter (49) entwässert, kaskadenförmig gespült und als Inert 4 (50) ausgetragen. Das Filtrat aus dem Vakuumbandfilter (51) wird zur weiteren Eindickung in einen Lamellenklärer (54) geführt.

Die organische Fraktion (42.2) wird nach Zugabe von Flockungshilfsmittel (43.1) mit einer Zentrifuge oder Kammerfilterpresse (43) entwässert. Optional kann die überwiegend organische Fraktion (48.2) aus dem Wendelscheider (48) direkt direkt dem Stoffstrom 42.2 vor der Entwässerung (43) zugeführt werden. Die entwässerte Organik 4 (44) wird dem Feststoffbunker Organik (72) zugeführt. Die Suspension Organik 4 (45) kann im Bypass als Kreislaufwasser oder zu Spülzwecken rückgeführt werden.

Das Zentrat 52 enthält noch gelöste Schwermetalle und wird direkt der weiteren Schwermetallfällung (56) zugeführt.

### 5. Stufe nassmechanische Trennung - Schwermetallabtrennunq (53)

In dem Filtrat (51) aus dem Vakuumbandfilter (49) und dem Filtrat bzw. Zentrat (52) aus der Entwässerung (43) sind gelöste Schwermetalle und noch an Tonmineralien gebundene Schwermetalle vorkonzentriert, die im weiteren Prozess als Konzentrat abgeschieden werden.

Zunächst wird die Suspension mit den Tonmineralen (51) im Lamellenklärer (54) unter Zugabe von Flockungshilfsmittel (54.1) geflockt und kann so im Lamellenklärer eingedickt werden. Der eingedickte Tonmineralschlamm bzw. die entwässerte Feinfraktion (54.3) wird durch Zudosierung von Schwefelsäure (54.2) in der Extraktion (54.4) auf einen ph-Wert von 1 bis 2,5, vorzugsweise 2, eingestellt. Der niedrige pH-Wert gewährleistet eine Auslösung von Schwermetallen aus den Tonmineralen. Tonminerale sind mit einem Ionenaustauscher vergleichbar und adsorbieren Schwermetalle bei hohen pH-Werten. Diese Adsorption ist bei niedrigen pH-Werten umkehrbar, jedoch benötigt die Desorption eine höhere Verweilzeit als die reine Einstellung des pH-Wertes. In der Extraktion (54.4) wird eine Desorption mit einer Verweilzeit von 0,5 bis 3 Stunden, vorzugsweise 1,5 Stunden erreicht. Anschließend werden die gelösten Schwermetalle durch Waschung und Entwässerung (54.4 und 55) vom Feststoff getrennt, wobei der pH-Wert durch Zudosierung von Natronlauge (55.1) vor der Entwässerung zur Verbesserung der Wirksamkeit des Flockungshilfsmittels (55.2) etwas angehoben werden kann. Da zwischen der pH-Wert Einstellung mit NaOH auf pH-Werte von 2 bis 5, vorzugsweise 3,5, und der Entwässerung kein Verweilzeitbehälter installiert ist und nur Sekunden bis zur Entwässerung vergehen, resorbieren die Schwermetalle nicht. Nach Zugabe von Flockungshilfsmittel (55.2) werden in der Entwässerung (55) die Schwermetalle, gelöst im Filtrat (55.3) von der Tonmineralfeststoff-Fraktion (59) getrennt. Das Filtrat (55.3) wird mit dem Zentrat (52) gemischt un der Schwerfällung (56) zugeführt. Die Schwermetalle werden durch Zugabe von schwefelhaltigem Biogas oder Natriumsulfid (56.2) als Schwermetallsulfide gefällt. Das schwefelhaltige Biogas (56.2) wird durch die Schwermetallfällung teilentschwefelt und kann in die Biogasreinigung zurückgeführt werden. In die Suspension mit den gefällten Schwermetallsulfiden ( 56.1 ) wird Flockungshilfsmittel zugegeben (57.), um Schwermetallsulfide mittel Kammerfilterpresse (57) als Schwermetallkonzentrat (58) abzuscheiden. Optional kann dieses Filtrat mit einem Ionenaustauscher nachbehandelt werden. Aber auch der direkte Einsatz eines Ionenaustauschers anstelle der Schwermetallfällung ist möglich.

Das gewonnene Filtrat (60) stellt das sauberste Wasser im System dar. Dieses Filtrat (60) kann zur Spülung der gewonnenen Produkte als Spülwasser (61) eingesetzt werden. Der Kreislaufwasserüberschuss (62) gelangt in die Vergärung.

### Niedertemperaturgarung (63)

Die Grobfraktionen Organik (27) > 4 mm bis > 12 mm, vorzugsweise 8 mm, sowie (26) > 25 mm bis > 50 mm, vorzugsweise > 35 mm werden gemeinsam oder einzeln in die Niedertemperaturgarung (64) automatisch eingetragen. Zur Hygienisierung werden die Fraktionen 26 und 27 bei einer Temperatur von 70°C bis 95°C, vorzugsweise 80°C, über mindestens 4 Stunden bis mindestens 12 Stunden, vorzugsweise mindestens 6 Stunden, in einem automatisch befüll- und entleerbaren Pendelbodenbunker (64) behandelt.

Die Temperatur und der pH-Wert im Bunker (64) werden durch Perkolation von Kreislaufwasser eingestellt und das Perkolat mittels Plattenwärmetauscher auf eine Temperatur von 65 °C bis 95 °C, vorzugsweise 80 °C, eingestellt. Der pH-Wert wird, durch Zugabe von Schwefelsäure zum Perkolat auf 4,5 bis 5,5, vorzugsweise pH 5, eingestellt wird.

Vorteilhafterweise beträgt die Verweilzeit im Bunker ca. 24 Stunden, um Papier, zellulosebasierte Materialien und Etikettenkleber durch Quellung und enzymatische Behandlung aufzulösen sowie um gleichzeitig eine Entkopplung der Betriebszeit der Befüllung und Entleerung der Bunker durch mindestens zwei Bunker zu erreichen. Das aus dem Bunker (64) ausgetragene Material wird mit einer Schneckenpresse (65) entwässert, durch eine Lösetrommel oder einen Konditioneur (66) zerfasert und vereinzelt sowie anschließend mit einem Spannwellensieb oder Sternsieb (67) bei 4 bis 12 mm, vorzugsweise 8 mm gesiebt.

Optional kann der Siebüberlauf (68) nachgesiebt werden bei > 25 mm bis > 60 mm, vorzugsweise > 35 mm. Die Mittelfraktion größer 4 - 12 mm, vorzugsweise 8 mm und kleiner 25 bis 60 mm, vorzugsweise 35 mm kann einer weiteren thermomechanische Zelllyse unterzogen werden, mittels Schneckenpresse bei höherem Pressdruck als bei der ersten Pressung (65). Die optional entstehende neue Grobfraktion > 25 mm bis > 60 mm, vorzugsweise > 35 mm wird Während der zusätzlichen Siebung mit Perkolat gespült und je nach Anforderungen der nachfolgenden Kunststoffaufbereitung (70), auf < 50 mm nachzerkleinert. Diese Korngröße ist speziell für eine nachfolgende Schwimm-Sink-Trennung erforderlich. Das überschüssige Perkolat bzw. Presswasser wird in den Hauptprozess zurückgeführt, um gelöste Mineralstoffe abzuscheiden. Die Siebfraktion kleiner 4 -12 mm, vorzugsweise kleiner 8 mm (69) wird dem Vorlagebunker (72) der Vergärung zugeführt.

### Kunststoffaufbereitunq (70)

Die Fraktion 68 gelangt in die Kunststoffaufbereitung (70), in der durch die unterschiedlichen Dichten der Kunststoffe durch eine mehrstufige Schwimm-Sink-Trennung die Kunststoffsorten vorgetrennt werden. Eine weitere Trennung in sortenreine Kunststoffe erfolgt unter Nutzung weiterer unterschiedlicher Kunststoffeigenschaften, wie unterschiedlicher elektrostatische Eigenschaften sowie unterschiedlicher aerodynamischer Eigenschaften (Windsichtung) und unterschiedlichem Reflexionsvermögen (Nahinfrarot, Röntgenstrahlung). Neben den Kunststoffen werden auch Faserstoffe, Holz, Leder und Metalle abgetrennt. Die Faserstoffe werden für die Produktion von faserverstärkten Kunststoffen verwendet.

### Vergärung (71)

In die Vergärung (71) gelangen die festen organischen Fraktionen 69 und 46 mit einem Trockensubstanzgehalt von 45% bis 60% im Gemisch. Die festen organischen Fraktionen haben einen pH-Wert von ca. 5 und eine Temperatur von größer 70 °C. Diese Festfraktion wird in den automatisch dosierenden Feststoffbunker (72) so eingetragen, dass eine Verweilzeit von mindestens einer Stunde bei 70 °C gewährleistet wird. Die Dosierung des Feststoffes in die Vergärung erfolgt mit Feststoffpumpen unter Rückmischung von pumpfähiger Suspension aus der Vergärung. Zusätzlich wird Überschusswasser (62) aus der nassmechanischen Trennung mit einer Temperatur größer als 70 °C der Flüssigvorlage (73) so zugeführt, dass eine Verweilzeit ohne Vermischung von mindestens einer Stunde gewährleistet ist. Zusätzlich können flüssige Gärsubstrate nach der Hygienisierung des Überschusswassers (62), die keine speziellen Hygienisierungsanforderungen haben, zugeführt werden. Dies sind zum Beispiel Gülle oder separat hygienisierte Speisereste oder Schlachthofabfälle. Die Vergärung ist als zweistufige Vergärung mit stehenden Behältern ausgeführt. Die Behälter (75 und 76) haben ein Durchmesser-HöhenVerhältnis von 1 zu 2 und werden als thermophile Pfropfenstromvergärung ausgeführt. Die Vergärung kann sowohl mit Trockensubstanzgehalten im Input von bis zu 60% Trockensubstanzgehalt betrieben werden als auch mit Flüssigkeiten, wie zum Beispiel Gülle. Die Strömungsrichtung erfolgt vom unteren Eintrag zum oberen Austrag. Die Rühraggregate sind als außenliegende MTS-Rührer ausgeführt und sind für Gärbehältergrößen bis zu 15.000 m³ geeignet. Die Rührung erfolgt nur im unteren Eintragsbereich als auch im oberen Austragsbereich. Somit ergibt sich in der Mitte ein aufwärtsgerichteter Pfropfenstrom. Somit ist eine Hygienisierung nach dem Stand der Technik schon alleine durch die Vergärung gewährleistet. Aufgrund des Einsatzes von Schwefelsäure in der Nassaufbereitung und Schwermetallabscheidung zur pH-Wert-Einstellung ist gerade bei einer thermophilen Fahrweise mit einer erhöhten Schwefelwasserstoffkonzentration in der Vergärung und im Biogas zu rechnen. Das Biogas (77) wird in einer Biogasentschwefelung mit Schwefelsäureproduktion vor der weiteren Biogasnutzung (78) gereinigt. Zur Senkung der Konzentration von Schwefelwasserstoff in der Vergärung kann ein Teilstrom des gereinigten Biogases zur Strippung von Schwefelwasserstoff zurückgeführt werden.

### Gärresteaufbereitunq (80)

Der Gärrest (79) aus der Vergärung hat eine Korngröße von 4 bis 12 mm, vorzugsweise 8 mm, und hat eine Teemperatur von ca. 50 bis 60 °C, im Mittel 55 °C, und einen pH-Wert von ca. 8 bis 8,5. Durch Zugabe von NaOH (81.1) wird der pH-Wert auf 10 bis 12, vorzugsweise 11, vor der Entwässerung mit einer Zentrifuge (81) erhöht. Dies hat den Vorteil, dass in der Zentrifuge mit dem Feststoff auch Kalk und Phosphat abgeschieden wird. Gleichzeitig wird der für die nachfolgende Ammoniakstrippung (88) erforderliche pH-Wert eingestellt. Vor der Entwässerung wird durch Rückführung des Zentrates (83) der Trockensubstanzgehalt des Gärrestes entsprechend den Anforderungen der Zentrifuge (81) reduziert. Nach dem Stand der Technik wird bei Entwässerung von Gärresten aus einer Trockenvergärung eine Schneckenpresse eingesetzt. Die Erfahrung mit Nassvergärungsanlagen bei Einsatz von Zentrifugen haben gezeigt, dass sich die Kunststoffe im Zentrat anreichern und der Gärrest somit weiter gereinigt wird und verwertbare Kunststoffe abgeschieden werden. Zentrifugenhersteller haben daher nach dem Stand der Technik Zentrifugen entwickelt, die Gärreste in die Fraktionen: mechanisch entwässerte Kunststofffraktion (82), den mechanisch entwässerten biogenen Gärrest (84) sowie das Zentrat (83) trennen. Aufgrund dieser Entwicklung wird bei dem erfindungsgemäßen Verfahren eine Zentrifuge (81) mit der Möglichkeit zur Kunststoffabscheidung eingesetzt. Zur Vermeidung eines Austrags von Phosphor in die Strippung und möglicher Verschmutzungen der Strippung wird das Zentrat (83) über eine Feinfilterung (85) gereinigt. Die festen Gärreste 84 und 86 werden in einem Bandtrockner (90) getrocknet. Als Zuluft der Trocknung wird die Abluft aus dem Bunker und den Aufbereitungshallen verwendet. Die Behandlung der Abluft aus dem Trockner (90.2) wird in einem mehrstufigen chemischen Wäscher (91) mit alkalischer und saurer Waschstufe gereinigt. In der alkalischen Waschstufe erfolgt eine chemische Oxidation von organischen Stoffen mittels Wasserstoffperoxid. Der getrocknete Gärrest (90.1) wird in einer Pelletierung (92) zu phosphorhaltigen Düngerpellets (92.1) verarbeitet. Die Pellets werden in Silos gelagert und können bedarfsgerecht mit weiteren Düngerkomponenten zu einem 1a Qualitätsdünger gemischt werden.

Das Filtrat (87) aus der Feinfilterung (85) hat die idealen Parameter von ca, 55 °C und pH-Wert 11 für die nachfolgende Strippung (88) zur Produktion von Ammoniumsulfat (88.2) als Stickstoffdünger. Sowohl der Stickstoffdünger als auch die Phosphorpellets stellen eine qualitätsgesicherte Handelsware mit Weltmarktwert dar, auch wenn diese Produkte aus Abfällen produziert wurden. Zusätzlich zum Stickstoff gelangt über die Zugabe von Schwefelsäure (88.1), die aus dem Abfall gewonnen wird, Schwefel als zusätzliche Düngerkomponente in den Stickstoffdünger. Durch die Maßnahmen der Abgasreinigung gelangt immer weniger Schwefel in die Landwirtschaft, so dass viele Böden bereits einen Schwefelmangel aufweisen. Sowohl die Ammoniakstrippung (88) als auch die Abluftbehandlung (91) verwenden das gleiche Chemikalienlager (89).

### Glas- und Metallrecyclinq (16)

Durch die nassmechanische Trennung werden die Vorprodukte 14 und 15 für das Glas-, Baustoff- und Metallrecycling mit Korngrößen größer 2 mm erzeugt. Ist das Vorprodukt hygienisiert und gewaschen erst einmal erzeugt, bestehen zahlreiche automatische, optische und andere Systeme zur 100prozentigen Verwertung dieser Stoffströme als Baustoffe, Glas und Metalle. Aus den Fraktionen 38 und 50 können nach einer Trocknung mittels magnetischer, elektrostatischer und Wirbelstrom-Trennung Eisenmetalle und Nicht-Eisenmetalle gewonnen werden. Die Fraktionen 38 und 50 sind vor, und besonders auch nach, der Metallabtrennung, als schadstoffarme und hochqualitative Baustoffe einsetzbar, da diese besonders kalkarm und salzarm sind. Die Fraktion 58 ist ein begehrter Rohstoff für die Metallhüttenindustrie, da diese Fraktion hohe Anteile an kritischen Elementen wie Edelmetalle und Seltenerdmetalle enthält.

### Ausführungsbeispiele

In einer besonderen Ausführung der Erfindung wird das gereinigte Filtrat nach der sulfidischen Fällung als Zusatzwasser für die Biogasentschwefelung verwendet.

In einer besonderen Ausführung der Erfindung werden die im Mischer (6) durch pH-Wert-Senkung und Temperaturerhöhung ausgasenden Geruchstoffe und Schadgase separat als Abluft erfasst und über einen alkalischen und sauren Wäscher (91) mit Einstellung des Redox-Potentials im alkalischen Wäscherbereich, geregelt über die Zugabe von Wasserstoffperoxid (89), oxidiert und damit die Abluft gereinigt und desodoriert. Im Mischer (6) wird vorrangig die Schwimmschicht zur besseren Ausgasung und Auflösung des Abfalls gemischt und durch mehrere schnelllaufende Schneckenförderer mit ca. 0,5 m bis 1,5 m, vorzugsweise 1 m, Flügelsteigung und einer Drehzahl von 150 bis 300 Umdrehungen pro Minute, die fliegend gelagert sind, mit einem Frequenzumrichter ausgestattet sind und zwei oder mehr Flügel aufweisen und von oben angetrieben werden.

In einer besonderen Ausführung der Erfindung wird die Suspension (8) erst nach einer ersten Abscheidung (12) von Schwerstoffen (14) bei > 5 mm bis > 50 mm, vorzugsweise > 35 mm, mit einem Sternsieb (13) gesiebt.

In einer besonderen Ausführung der Erfindung wird die Suspension (21) nach der ersten Siebung (13) nach einer weiteren Schwerstoffabscheidung (23) bei 4 mm bis 15 mm, vorzugsweise bei 8 mm, mit einem Spannwellensieb (24) gesiebt.

In einer besonderen Ausführung der Erfindung werden zur Einstellung der Strömungsgeschwindigkeiten bei den Schwerstoffabscheidungen (12 und 23) ein oder mehrere schnelllaufende Schneckenförderer mit einer Flügelsteigung von ca. 0,5 m bis 1,5 m, vorzugsweise 1 m, und einer Drehzahl von 150 bis 300 Umdrehungen pro Minute, die fliegend gelagert sind, mit einem Frequenzumrichter ausgestattet sind und zwei oder mehr Flügel aufweisen sowie von oben angetrieben werden, eingesetzt.

In einer besonderen Ausführung der Erfindung wird die Suspension (39) < 50 µm bis < 150 µm, vorzugsweise < 100 µm, durch Kavitation oder durch Ultraschall (47) vor der Reinigung mittels Wendelscheider (48) vorbehandelt.

In einer besonderen Ausführung der Erfindung **wird** die mineralische Fraktion (48.1) < 50 µm bis 150 µm, vorzugsweise < 100 µm mittels Vakuumbandfilter (49) entwässert.

In einer besonderen Ausführung der Erfindung wird die gewaschene und von Schluffanteilen und Schwermetallen gereinigte und mechanisch entwässerte Feinstfraktion (59) zur Einstellung der Dichte bei einer Schwimm-Sink-Trennung der Kunststoffgemischfraktionen eingesetzt.

In einer besonderen Ausführung der Erfindung werden einzelne oder auch alle organischen festen Fraktionen (34,44, 59 und 69) und Kreislaufwasser (62) nach der Hygienisierung durch Lagerung bei > 70°C über mehr als eine Stunde einer Vergärung zugeführt, die am Standort der Aufbereitung oder einem anderen Standort durchgeführt wird. Die Vergärung ist vorzugsweise
thermophil und ein- oder zweistufig, vorzugsweise zweistufig ausgeführt.

In einer besonderen Ausführung der Erfindung wird der Gärrest (79) vor der Entwässerung mittels Zentrifuge (81) durch Dosierung von Natronlauge (81.1) auf einen pH-Wert von 10 - 12, vorzugsweise 11, eingestellt und so viel Zentrat (83) zu der Zentrifuge (81) zurückgeführt, dass der für die Zentrifuge optimale Trockensubstanzgehalt eingestellt werden kann und somit die Zentrifuge neben der Funktion der Entwässerung des Gärrestes inkl. des gefällten Phosphates auch separat leichte Kunststoffe wie PP, PE, Styropor oder Kunststoffe ähnlicher Dichte < 1 kg/l abscheiden kann.

In einer besonderen Ausführung der Erfindung wird aus dem Zentrat (83) nach einer Feinfilterung bei < 5 µm bis 100 µm, vorzugsweise < 20 µm mit z.B. einem Bandfilter oder einer Kammerfilterpresse (85) Ammoniumsulfat als Dünger mittels einer Strippungsanlage (88) gewonnen.

In einer besonderen Ausführung der Erfindung wird die Grobfraktionen (27) > 4 mm bis > 12 mm, vorzugsweise 8 mm, sowie (26) > 25 mm bis > 50 mm, vorzugsweise > 35 mm, gemeinsam oder einzeln zur Niedertemperaturgarung (64) und zur Hygienisierung bei einer Temperatur von 70°C bis 95°C, vorzugsweise 80°C, über mindestens 4 Stunden bis mindestens 12 Stunden, vorzugsweise mindestens 6 Stunden, in einem automatisch befüll- und entleerbaren Pendelbodenbunker (64) behandelt.,

In einer besonderen Ausführung der Erfindung wird das Kreislaufwasser mit Säure (10) so gemischt, dass ein pH-Wert der Suspension (8) von 4 bis 6, vorzugsweise pH-Wert 5 eingestellt wird.

In einer besonderen Ausführung der Erfindung werden die Temperatur und der pH-Wert im Bunker (64) durch Perkolation von Kreislaufwasser eingestellt und das Perkolat mittels Plattenwärmetauscher auf eine Temperatur von 65 °C bis 95 °C, vorzugsweise 80 °C, eingestellt und der pH-Wert auf 4,5 bis 5,5, vorzugsweise pH 5, durch Zugabe von Schwefelsäure zum Perkolat eingestellt,

In einer besonderen Ausführung der Erfindung beträgt die Verweilzeit im Bunker (64) ca. 24 Stunden, um Papier, zellulosebasierte Materialien und Etikettenkleber durch Quellung und enzymatische Behandlung aufzulösen sowie um gleichzeitig eine Entkopplung der Betriebszeit der Befüllung und Entleerung der Bunker durch mindestens zwei Bunker zu erreichen.

In einer besonderen Ausführung der Erfindung wird das aus dem Bunker (64) ausgetragene Material mit einer Schneckenpresse (65) entwässert, durch eine Lösetrommel oder einen Konditioneur (66) zerfasert und vereinzelt sowie anschließend mit einem Spannwellensieb oder Sternsieb (67) gesiebt.

In einer besonderen Ausführung der Erfindung wird die Siebung (67) zweistufig bei > 25 mm bis > 60 mm, vorzugsweise > 35 mm, sowie zusätzlich bei > 4 mm bis > 12 mm, vorzugsweise 8 mm, durchgeführt.
In einer besonderen Ausführung der Erfindung wird die Grobfraktion (68) > 25 mm bis > 60 mm, vorzugsweise > 35 mm nachzerkleinert auf < 50 mm.

In einer besonderen Ausführung der Erfindung wird während einer Siebung mit Perkolat gespült und eine Fraktion > 25 mm bis > 50 mm, vorzugsweise > 35 mm, wieder einem Bunker mit temperierter Perkolation zugeführt sowie mit einer Fraktion < 25 mm bis < 50 mm, vorzugsweise < 35 mm, eine weitere thermomechanische Zelllyse mittels Schneckenpresse bei höherem Pressdruck als bei der ersten Pressung (65) durchgeführt.,

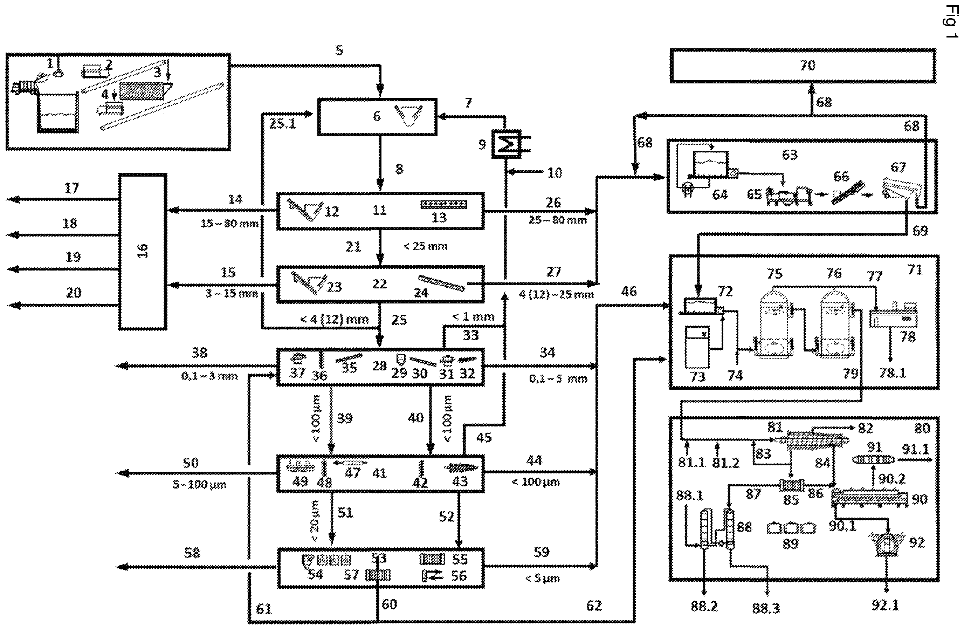

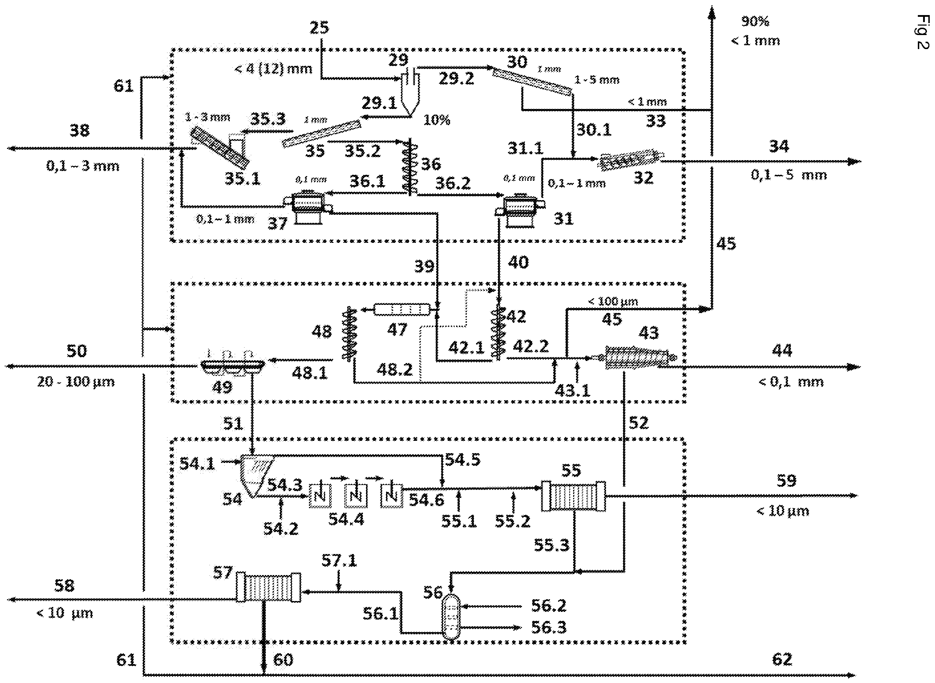

### Legende Fig. 1 und Fig. 2

| | |
|---|---|
| 1 | Bunker |
| 2 | Vorzerkleinerer |
| 3 | Siebtrommel 80 mm |
| 4 | Nachzerkleinerer < 80 mm |
| 5 | Abfall < 80 mm |
| 6 | Mischer |
| 7 | heißes saures Kreislaufwasser |
| 8 | Abfallsuspension < 80 mm |
| 9 | Wärmetauscher Kreislaufwasser |
| 10 | Dosierung Schwefelsäure aus der Biogasreinigung |
| 11 | 1. Stufe Nassmechanische Trennung |
| 12 | Abscheidekammer 1 - Steinabscheidung |
| 13 | Sternsieb 25 - 35 mm, Alternativ Spannwellensieb |
| 14 | Inert 1 - Steine 15 - 80 mm vor der automatischen Sortierung |
| 15 | Inert 2 - Kies 3 - 15 mm vor der automatischen Sortierung |
| 16 | Glas-Metall-Sortierung |
| 17 | Glas nach Farben sortiert |
| 18 | verschiedene Recyclingbaustoffe |
| 19 | Eisen |
| 20 | Nichteisenmetalle |
| 21 | Abfallsuspension < 25 mm |
| 22 | 2. Stufe Nassmechanische Trennung |
| 23 | Abscheidekammer 2 - Kiesabscheidung |
| 24 | Spannwellensieb 4 mm bis 12 mm, vorzugsweise < 8 mm |
| 25 | Abfallsuspension < 4 mm bis < 12 mm, vorzugsweise < 8 mm |
| 25.1 | Rückführung Abfallsuspension < 4 mm bis < 12 mm |
| 26 | Organik 1 - Organik-Gemisch (Biomasse und Kunststoffe) 25 (35) mm bis 80 mm |
| 27 | Organik 2 - Organik-Gemisch (Biomasse und Kunststoffe) 4 (12) mm bis 25 (35) mm |
| 28 | 3. Stufe Nassmechanische Trennung |
| 29 | Hydrozyklon Abscheidung Sand > 1 mm |
| 29.1 | Hydrozyklonunterlauf |
| 29.2 | Hydrozyklonüberlauf |
| 30 | Spannwellensieb 1 (1,5) mm |
| 30.1 | Organik 3a |
| 31 | Vibrationssieb Organik 100 µm bis 300 µm |
| 31.1 | Organik 3b |
| 32 | Schneckenpresse Organik 3 |
| 33 | Kreislaufwasser < 1 mm zum Wärmetauscher |
| 34 | Pressgut Organik 3 (0,1 - 5 (10) mm) zum Feststoffbunker Vergärung |
| 35 | Spannwellensieb 1 mm zur Absiebung Inert 3.1 zum Aufstromklassierer Sand 35.1 |
| 35.1 | Abscheidekammer 3 - Sandabscheidung |
| 35.2 | Siebunterlauf < 0,75 - 1,5 mm, vorzugsweise < 1 mm |
| 35.3 | Siebüberlauf >0,75 - 1,5 mm, vorzugsweise > 1 mm und < 4 bis <12 mm, vorzugsweise < 8 mm |
| 36 | Wendelscheider 1 zur Trennung < 1 mm |
| 36.1 | Inertsuspension aus Wendelscheider 1 |
| 36.2 | Organiksuspension aus Wendelscheider 1 |
| 37 | Vibrationssieb Inert 3.2 mit der Korngröße 100 µm (300 µm) bis 1 mm (1,5 mm) |
| 38 | Inert 3 mit der Korngröße 100 µm (300 µm) bis ca. 3 mm |
| 39 | Suspension Inert 4 < 100 µm (300 µm) |
| 40 | Suspension Organik 4 < 100 µm (300 µm) |
| 41 | 4. Stufe Nassmechanische Trennung |
| 42 | Wendelscheider 2a zur Reinigung Organik 4 < 100 (300) µm |
| 42.1 | Inertsuspension aus Wendelscheider 2a |
| 42.2 | Organiksuspension aus Wendelscheider 2a |
| 43 | Zentrifuge oder Kammerfilterpresse zur Entwässerung der Organik 4 der 4. Stufe Nassmechanische Trennung |
| 43.1 | Flockungshilfsmittel |
| 44 | Organik 4 entwässert zum Feststoffbunker Vergärung |
| 45 | Bypass Suspension Organik 4 als Kreislaufwasser zur Rückführung |
| 46 | Gemisch aus mechanisch entwässerter Organik 3, Organik 4 und Tonmineral zur Vergärung |
| 47 | Ultraschall zur Reinigung Schluffpartikel durch Kavitation |
| 48 | Wendelscheider 2b zur Reinigung Inert < 100 (300) µm |
| 48.1 | Inertsuspension aus Wendelscheider 2b |
| 48.2 | Organiksuspension aus Wendelscheider 2b |
| 49 | Vakuumbandfilter zur Spülung und Entwässerung der Schlufffraktion Inert 4 |
| 50 | Inert 4 - Schluff 5 (20) µm bis 100 (300) µm |
| 51 | Filtrat Vakuumbandfilter |
| 52 | Zentrifugat Zentrifuge Entwässerung Organik 4 |
| 53 | 5. Stufe Tonmineralregeneration und Schwermetallabscheidung |
| 54 | Lamellenklärer |
| 54.1 | Dosierung Flockungshilfsmittel |
| 54.2 | Zugabe Schwefelsäure zur Einstellung eines für die Regeneration erforderlichen sehr niedrigen pH-Wertes < 2,5 bis < 1,5 |
| 54.3 | eingedickte Tonmineralsuspension |
| 54.4 | Extraktionsbehälter zur sauren Tonmineralregeneration |
| 54.5 | Klarlauf Lamellenklärer |
| 54.6 | regenerierte Tonmineralsuspension pH-Wert < 2,5 bis < 1,5 |
| 55 | Kammerfilterpresse |
| 55.1 | NaOH-Dosierung zur Anhebung des pH-Wertes auf ca. pH 5 in Abhängigkeit der Säuretoleranz des Flockungshilfsmittels |
| 55.2 | Dosierung Flockungshilfsmittel |
| 55.3 | Filtrat Kammerfilterpresse |
| 56 | Sulfidische Schwermetallfällung mit Schwefelwasserstoff aus dem Biogas. Alternativ Zugabe von Natriumsulfid. |
| 56.1 | Suspension nach der Schwermetallfällung |
| 56.2 | schwefelhaltiges Biogas |
| 56.3 | teilentschwefeltes Biogas |
| 57 | Kammerfilterpresse zur Entwässerung des Schwermetallkonzentrates |
| 57.1 | Zugabe von Flockungshilfsmittel zur Flockung von Schwermetallsulfid |
| 58 | Schwermetallkonzentrat |
| 59 | Regeneriertes Tonmineral zur Vergärung |
| 60 | Filtrat Kammerfilterpresse Entwässerung Schwermetallkonzentrat |
| 61 | Filtrat Kammerfilterpresse Schwermetall zur Spülung von Organik und Inert in Stufe 3 und Stufe 4 |
| 62 | Filtrat Kammerfilterpresse Schwermetall zur Vergärung |
| 63 | Zelllyse - Anreichung der Kunststoff/Holz-Fraktion und Gewinnung der kunststoffarmen Biomassefraktion |
| 64 | Niedertemperaturkocher, ausgeführt als Betontunnel mit Pendelbodensystem. Durch Perkolation wird eine Aufheizung und Niedertemperaturkochung bei 80°C ermöglicht. |
| 65 | Schneckenpresse bei > 70°C bewirken eine Zerfaserung der organischen Fraktion |
| 66 | Konditioneur zur Auflösung vor der Siebung |
| 67 | Siebung mindestens bei 5 (10 ) mm mittels Sternsieb oder Spannwellensieb. Zusätzlich kann eine Doppelsiebung bei 30 (40) mm für die nachfolgende Kunststoffaufbereitung vorteilhaft sein |
| 68 | Siebüberlauf zur Kunststoffaufbereitung |
| 69 | Siebunterlauf < 5 (10) mm zur Vergärung |
| 70 | Kunststoffaufbereitung |
| 71 | Vergärung |
| 72 | Feststoffbunker Vergärung |
| 73 | Suspensionsvorlage Vergärung |
| 74 | Flüssige Substrate wie z.B. Gülle |
| 75 | Hauptgärer |
| 76 | Nachgärer |
| 77 | Biogas |
| 78 | Biogasreinigung und Biogasnutzung als BHKW oder Biomethanaufbereitung |
| 78.1 | Schwefelsäure aus der Biogasentschwefelung |
| 79 | Gärrest |
| 80 | Gärresteaufbereitung |
| 81 | Zentrifuge Entwässerung Gärrestemit Abscheidung und Entwässerung der leichten Kunststofffraktion PP, PE und Styropor etc. |
| 81.1 | Dosierung Natronlauge zur Einstellung pH-Wert von ca. 11 zur Fällung des gelösten Phosphates |
| 81.2 | Dosierung Flockungshilfsmittel |
| 82 | Entwässerte Restkunststoffe vorrangig PP, PE und Styropor |
| 83 | Rückführung Zentrat zur Einstellung des Trockensubstanzgehaltes vor der Zentrifuge |
| 84 | Entwässerter Gärrest von Restkunststoffen befreit |
| 85 | Feinfilterung Zentrat durch Kammerfilterpresse oder ähnliches |
| 86 | Entwässerter Feinschlamm aus Feinfilterung |
| 87 | Filtrat mit pH-Wert ca. 11 und Temperatur ca. 50 - 55°C |
| 88 | Strippung Filtrat |
| 88.1 | Zugabe Schwefelsäure aus der Biogasreinigung oder andere Schwefelsäure |
| 88.2 | Ammoniumsulfat |
| 88.3 | Flüssigdünger Kalium und Spurenelemente |
| 89 | Chemikalienlager Natronlauge, Schwefelsäure und Wasserstoffperoxyd für die Strippung, Einstellung pH-Werte Gesamtanlage und Abluftreinigung |
| 90 | Gärrestetrockner |
| 90.1 | Getrocknete Gärreste |
| 90.2 | Abluft Gärrestetrockner |
| 91 | Abluftreinigung ausgeführt als mehrstufiger chemischer Wäscher |
| 91.1 | gereinigte Abluft |
| 92 | Pelletierung |
| 92.1 | Pellets mit hohem Phosphat- und Biomassegehalt |

## Patentansprüche

1. Verfahren zur Schadstoffreduzierung von Abfällen mit über 20% biogenem Anteil, der überwiegend leicht biologisch anaerob abbaubar ist und von Feststoffen, die aus Abwasser oder flüssigen Abfällen abgesiebt sind und einen biogenen Anteil von über 20% aufweisen, der überwiegend leicht biologisch anaerob abbaubar ist,
**dadurch gekennzeichnet, dass**
durch Waschung und Siebung sowie anschließende Filterung und mechanische Entwässerung, jedoch ohne vorherige biologische Behandlung und ohne Rückführung von Presswasser aus der Vergärung, eine Feinfraktion erzeugt wird, dass aus dieser Feinfraktion durch Versäuerung mit Schwefelsäure Schwermetalle ausgewaschen werden, dass diese Schwermetalle durch sulfidische Fällung abgeschieden werden und erst die gereinigte Feinfraktion zur Biogasproduktion und Rückgewinnung der Schwefelsäure anaerob biologisch behandelt wird.

2. Verfahren nach Anspruch 1, **dadurch gekennzeichnet, dass** Abfälle zur Erzeugung der Feinfraktion mit Kreislaufwasser (25.1 und 7) zu einer Suspension (8) gemischt und danach getrennt werden, und dass eine schadstoffhaltige Feinfraktion < 100 µm bis < 1.000 µm, vorzugsweise < 500 µm (39 bzw. 51), entwässert wird und diese entwässerte Feinfraktion mehrstufig im Gegenstrom mit Schwefelsäurehaltigem Waschwasser gewaschen und mehrstufig entwässert wird.

3. Verfahren nach Anspruch 1 und 2, **dadurch gekennzeichnet, dass** durch Vorwärmung des Kreislaufwassers (7) eine Temperatur der Suspension (8) von 65 °C bis 95 °C, vorzugsweise 75 °C, eingestellt wird.

4. Verfahren nach Anspruch 1, 2 und 3, **dadurch gekennzeichnet, dass** nur die Suspension (33), die zur Einhaltung der Prozesstemperatur erforderlich ist, über den Wärmetauscher (9) geleitet wird und vorher mittels Zyklon (29) von Mineralstoffen > 0,5 mm bis > 1,5 mm, vorzugsweise > 1 mm, befreit wird und danach über ein Spannwellensieb (30) bei 1 mm bis 2 mm, vorzugsweise 1,5 mm, abgesiebt wird.

5. Verfahren nach Anspruch 1 und 4, **dadurch gekennzeichnet dass** die Regelung der Kreislaufwassermenge, die für die Einstellung der Feststoffkonzentration der Mischung (8) mit dem Abfall (5) erforderlich ist, maßgeblich durch die Rückführung der Suspension < 4 mm bis < 12 mm, vorzugsweise < 8 mm (25.1), erfolgt.

6. Verfahren nach Anspruch 1 und 4 , **dadurch gekennzeichnet, dass** nur der Zyklonunterlauf (29.1) zur weiteren Aufbereitung der Feinsuspension < 1 mm bis < 2 mm, vorzugsweise < 1,5 mm (35.2) verwendet wird und sich dadurch die Feinstoffe < 1 mm bis < 2 mm, vorzugsweise < 1,5 mm, die in den Zyklonüberlauf (29.2) gelangen und nicht ausgeschleust wurden, sich im Kreislaufwasser (7, 8, 21 und 25) bis zu einem Gleichgewichtszustand anreichern.

7. Verfahren nach Anspruch 1 und 6, **dadurch gekennzeichnet, dass** der Gleichgewichtszustand der Anreicherung von Feinstoffen < 1 mm bis < 2 mm, vorzugsweise < 1,5 mm durch Veränderung der Überlauf- und Unterlaufquerschnitte des Hydrozyklons (29) verändert wird und die Dichte und damit das Trennverhalten der vorgelagerten Prozessschritte geregelt wird.

8. Verfahren nach Anspruch 1, 4 und 6, **dadurch gekennzeichnet, dass** der Zyklonunterlauf (29.1) auf < 1 mm bis < 2 mm, vorzugsweise < 1,5 mm, mit einem Spannwellensieb (35) gesiebt wird und der Siebüberlauf > 1 mm bis > 2 mm, vorzugsweise > 1,5 mm (35.3), und < 4 mm bis < 12 mm, vorzugsweise < 8 mm, mit einem Aufstromklassierer (35.1) von Faserstoffen gereinigt wird.

9. Verfahren nach Anspruch 1 und 8, **dadurch gekennzeichnet, dass** der auf < 1 mm bis < 2 mm, vorzugsweise < 1,5 mm gesiebte Zyklonunterlauf (35.2) über einen Wendelscheider (36) in eine organische (36.2) und eine mineralische (36.1) Fraktion getrennt wird.

10. Verfahren nach Anspruch 1 und 9, **dadurch gekennzeichnet, dass** die organische Fraktion (36.2) mit einem Vibrationssieb (31) bei 50 µm bis 500 µm, vorzugsweise 100 µm, gesiebt wird.

11. Verfahren nach Anspruch 1, **dadurch gekennzeichnet, dass** von der Feinfraktion (40) < 50 µm bis 500 µm, vorzugsweise < 100 µm, eine SchluffFraktion (42.1) > 5 µm bis > 40 µm, vorzugsweise > 20 µm und < 50 µm bis 500 µm, vorzugsweise < 100 µm, mit einem Wendelscheider (42) vor der Entwässerung (43) abgeschieden wird.

12. Verfahren nach Anspruch 1 und 9, **dadurch gekennzeichnet, dass** die organische (36.2) und die mineralische (36.1) Fraktion aus dem Wendelscheider (36) nach Anspruch 27 jeweils separat auf < 50 µm bis < 300 µm, vorzugsweise < 100 µm, gesiebt werden (31 und 37) und der Siebdurchlauf (39 und 40) nochmals jeweils separat mit einem Wendelscheider (42 und 48) gereinigt wird, wobei jeweils eine überwiegend mineralische Fraktion (42.1 und 48.1) sowie jeweils eine überwiegend organische Fraktion (42.2 und 48.2) entstehen, und der mineralische Anteil aus dem Wendelscheider (42) der überwiegend organischen Fraktion (40) dem Wendelscheider (48) der mineralischen Fraktion (39) gemeinsam mit der überwiegend mineralischen Fraktion (42.1) zugegeben wird und dies kreuzweise auch mit der organischen Fraktion (48.2) aus dem, mit der überwiegend mineralischen Fraktion betriebenen Wendelscheider (48) durchgeführt wird.

13. Verfahren nach Anspruch 1 und 12, **dadurch gekennzeichnet, dass** die mineralische Fraktion (50) > 50 µm bis > 300 µm, vorzugsweise > 100 µm und die Fraktion (38) 100 µm bis 3 mm getrocknet wird und mittels magnetischer, elektrostatischer und Wirbelstrom-Trennung Eisenmetalle und Nicht-Eisenmetalle abgeschieden werden.

14. Verfahren nach Anspruch 1 und 9, **dadurch gekennzeichnet, dass** von der Suspension (35.2) < 0,5 mm bis 1,5 mm, vorzugsweise 1 mm, durch einen vorgeschalteten Wendelscheider (36) die Sand-Fraktion (36.1) > 50 µm bis > 150 µm, vorzugsweise > 100 µm, abgeschieden und mittels Vibrationssieb (37) gesiebt wird und über den Siebdurchlauf die danach abzuscheidende SchluffFraktion (39) vorkonzentriert wird.

## Claims

1. Method for reducing pollutants from waste with over 20% biogenic content that is predominantly easily biodegradable anaerobically, and of solids that are sieved from wastewater or liquid waste and have a biogenic content of over 20%, which is predominantly easily biodegradable anaerobically,
**characterized in that**
through washing and sieving as well as subsequent filtering and mechanical dewatering, but without prior biological treatment and without recycling press water from the fermentation, a fine fraction is produced, that heavy metals are washed out of this fine fraction by acidification with sulphuric acid, and that these heavy metals are separated by sulfidic precipitation and only the purified fine fraction is treated anaerobically biologically for biogas production and recovery of sulphuric acid.

2. Method according to claim 1, **characterized in that** waste for production of the fine fraction with circulating water (25.1 and 7) are mixed to suspension (8) and then separated, and that a pollutant-containing fine fraction <100 µm to <1,000 µm, preferably <500 µm (39 or 51), is dewatered and this dewatered fine fraction is washed in several stages in countercurrent with washing water containing sulphuric acid and dewatered in several stages.

3. Method according to claims 1 and 2, **characterized in that** a temperature of the suspension (8) of 65°C to 95°C, preferably 75°C, is set by preheating the circulating water (7).

4. Method according to claim 1, 2 and 3, **characterized in that** only the suspension (33), which is required to maintain the process temperature, is passed over the heat exchanger (9) and minerals > 0.5 mm to > 1.5 mm, preferably > 1 mm are removed by a cyclone (29), and the suspension is then screened over a flip-flop sieve (30) at 1 mm to 2 mm, preferably 1.5 mm.

5. Method according to claims 1 and 4, **characterized in that** the regulation of the amount of circulating water required for adjusting the solids concentration of the mixture (8) with the waste (5) is largely achieved by returning the suspension <4 mm to <12 mm, preferably < 8 mm (25.1).

6. Method according to claim 1 and 4, **characterized in that** only the cyclone underflow (29.1) is used for further processing of the fine suspension <1 mm to <2 mm, preferably <1.5 mm (35.2) and thereby the fines <1 mm to <2 mm, preferably <1.5 mm, which are in the cyclone overflow (29.2) and have not been discharged accumulate in the circulating water (7, 8, 21 and 25) to an equilibrium state.

7. Method according to claims 1 and 6, **characterized in that** the equilibrium state of enrichment of fines <1 mm to <2 mm, preferably <1.5 mm by changing the overflow and the underflow cross section of the hydrocyclone (29) is changed and the density and thus the separation behaviour of the upstream process steps is regulated.

8. Method according to claim 1, 4 and 6, **characterized in that** the cyclone underflow (29.1) is screened to <1 mm to <2 mm, preferably <1.5 mm, with a flip-flop sieve (35) and the sieve overflow >1 mm to >2 mm, preferably >1.5 mm (35.3), and <4 mm to <12 mm, preferably <8 mm, is cleaned of fiber materials using an upflow classifier (35.1).

9. Method according to claims 1 and 8, **characterized in that** the cyclone underflow (35.2), sieved to <1 mm to <2 mm, preferably <1.5 mm, is separated into an organic (36.2) and a mineral (36.1) fraction via a spiral separator (36).

10. Method according to claims 1 and 9, **characterized in that** the organic fraction (36.2) is sieved with a vibrating sieve (31) at 50 µm to 500 µm, preferably 100 µm.

11. Method according to claim 1, **characterized in that** a silt fraction (42.1) >5 µm to >40 µm, preferably >20 µm and <50 µm to 500 µm, preferably <100 µm, is separated from the fine fraction (40) <50 µm to 500 µm, preferably <100 µm, by a spiral separator (42) before dewatering (43).

12. Method according to claims 1 and 9, **characterized in that** the organic (36.2) and the mineral (36.1) fraction from the spiral separator (36) according to claim 11 are each separately screened to <50 µm to <300 µm, preferably <100 µm (31 and 37) and the sieve passage (39 and 40) is cleaned again separately with a spiral separator (42 and 48) with a predominantly mineral fraction (42.1 and 48.1) and a predominantly organic fraction (42.2 and 48.2) being created, and the mineral portion from the spiral separator (42) and the predominantly organic fraction (40) are added to the spiral separator (48) of the mineral Fraction (39) together with the predominantly mineral fraction (42.1) and this also crosswise is carried out with the organic fraction (48.2) from the predominantly mineral fraction operated spiral separator (48).

13. Method according to claims 1 and 12, **characterized in that** the mineral fraction (50) > 50 µm to > 300 µm, preferably > 100 µm and the fraction (38) 100 µm to 3 mm is dried and ferrous metals and non-ferrous metals are separated by means of magnetic, electrostatic and eddy current separation.

14. Method according to claims 1 and 9, **characterized in that** the sand fraction (36.1) >50 µm to >150 µm, preferably >100 µm is separated by an upstream spiral separator (36) from the suspension (35.2) <0.5 mm to 1.5 mm, preferably 1 mm, using a vibrating sieve (37), and the silt fraction (39) to be separated is pre-concentrated via the sieve pass.

## Revendications

1. Procédé de réduction des polluants provenant de déchets ayant une teneur biogénique supérieure à 20 %, qui est en grande partie facilement biodégradable en anaérobie, et de matières solides qui sont tamisées à partir d'eaux usées ou de déchets liquides et présentent une teneur biogénique supérieure à 20 %, qui est en grande partie facilement biodégradable en anaérobie,
**caractérisé en ce que**
par lavage et tamisage ainsi que par filtration et déshydratation mécanique ultérieures, mais sans traitement biologique préalable et sans recyclage de l'eau de presse issue de la fermentation, on obtient une fraction fine, que des métaux lourds sont éliminés de cette fraction fine par acidification avec de l'acide sulfurique, que ces métaux lourds sont séparés par précipitation sulfurique et que seule la fraction fine purifiée est traitée biologiquement en anaérobie pour la production de biogaz et la récupération de l'acide sulfurique.

2. Procédé selon la revendication 1, **caractérisé en ce que**, pour produire la fraction fine, des déchets sont mélangés avec de l'eau en circulation (25.1 et 7) pour former une suspension (8) et sont ensuite séparés, et qu'une fraction fine contenant des polluants < 100 µm à < 10000 µm, de préférence < 500 µm (39 ou bien 51), est déshydratée, et cette fraction fine déshydratée est lavée en plusieurs étapes à contre-courant avec de l'eau de lavage contenant de l'acide sulfurique et est déshydratée en plusieurs étapes.

3. Procédé selon la revendication 1 et 2, **caractérisé en ce qu'**une température de la suspension (8) comprise entre 65 °C et 95 °C, de préférence de 75 °C, est réglée par préchauffage de l'eau en circulation (7).

4. Procédé selon les revendications 1, 2 et 3, **caractérisé en ce que** seule la suspension (33) qui est nécessaire au maintien de la température de processus passe par l'échangeur thermique (9) et est préalablement libérée, au moyen d'un cyclone (29), de minéraux compris entre > 0,5 mm et > 1,5 mm, de préférence > 1 mm , et est ensuite tamisée sur un tamis à arbres tendeurs (30) à 1 mm à 2 mm, de préférence à 1,5 mm.

5. Procédé selon les revendications 1 et 4, **caractérisé en ce que** la régulation de la quantité d'eau en circulation qui est nécessaire au réglage de la concentration en matières solides du mélange (8) avec les déchets (5) se fait en grande partie par le recyclage de la suspension < 4 mm à <12 mm, de préférence <8 mm (25,1).

6. Procédé selon les revendications 1 et 4, **caractérisé en ce que** seule la sousverse du cyclone (29.1) est utilisée pour le traitement ultérieur de la suspension fine <1 mm à < 2 mm, de préférence <1,5 mm (35.2) et que, ainsi, les matières fines < 1 mm à < 2 mm, de préférence < 1,5 mm, qui entrent dans la surverse du cyclone (29.2) et n'ont pas été évacués, s'accumulent dans l'eau en circulation (7, 8, 21 et 25) jusqu'à un état d'équilibre.

7. Procédé selon les revendications 1 et 6, **caractérisé en ce que** l'état d'équilibre de l'enrichissement de matières fines < 1 mm à < 2 mm, de préférence < 1,5 mm, est modifié en modifiant les sections de surverse et de sousverse de l'hydrocyclone (29) et que la densité et donc le comportement de séparation des étapes de processus en amont sont régulés.

8. Procédé selon les revendications 1, 4 et 6, **caractérisé en ce que** la sousverse du cyclone (29.1) est tamisée à < 1 mm à < 2 mm, de préférence < 1,5 mm, au moyen d'un tamis à arbres tendeurs (35) et que la surverse de tamis > 1 mm à > 2 mm, de préférence > 1,5 mm (35.3), et < 4 mm à < 12 mm, de préférence < 8 mm, est nettoyée de matériaux fibreux au moyen d'un classificateur à flux ascendant (35.1).

9. Procédé selon les revendications 1 et 8, **caractérisé en ce que** la sousverse du cyclone (35.2) qui est tamisée à < 1 mm à < 2 mm, de préférence < 1,5 mm, est séparée par un séparateur en spirale (36) en une fraction organique (36.2) et une fraction minérale (36.1).

10. Procédé selon les revendications 1 et 9, **caractérisé en ce que** la fraction organique (36.2) est tamisée au moyen d'un tamis vibrant (31) à 50 µm à 500 µm, de préférence 100 µm.

11. Procédé selon la revendication 1, **caractérisé en ce qu'**une fraction limoneuse (42.1) > 5 µm à > 40 µm, de préférence > 20 µm et < 50 µm à 500 µm, de préférence < 100 µm, est séparée de la fraction fine (40) < 50 µm à 500 µm, de préférence < 100 µm, au moyen d'un séparateur en spirale (42) avant la déshydratation (43).

12. Procédé selon les revendications 1 et 9, **caractérisé en ce que** les fractions organique (36.2) et minérale (36.1) issues du séparateur en spirale (36) selon la revendication 11 sont tamisés (31 et 37) chacune séparément à < 50 µm à < 300 µm, de préférence < 100 µm, et le passage du tamis (39 et 40) est à nouveau nettoyé respectivement séparément au moyen d'un séparateur en spirale (42 et 48), dans lequel respectivement une fraction majoritairement minérale (42.1 et 48.1) ainsi que respectivement une fraction majoritairement organique (42.2 et 48.2) sont produites, et la partie minérale du séparateur en spirale (42) de la fraction majoritairement organique (40) est ajoutée au séparateur en spirale (48) de la fraction minérale (39) conjointement avec la fraction majoritairement minérale (42.1), et ceci est effectué également en croix avec la fraction organique (48.2) du séparateur en spirale (48) que l'on fait fonctionner avec la fraction majoritairement minérale.

13. Procédé selon les revendications 1 et 12, **caractérisé en ce que** la fraction minérale (50) > 50 µm à > 300 µm, de préférence > 100 µm et la fraction (38) 100 µm à 3 mm est séchée et que des métaux ferreux et des métaux non ferreux sont séparés au moyen d'une séparation magnétique, électrostatique et par courants de Foucault.

14. Procédé selon les revendications 1 et 9, **caractérisé en ce que** la fraction de sable (36.1) > 50 µm bis > 150 µm, de préférence > 100 µm, est séparée de la suspension (35.2) < 0,5 mm à 1,5 mm, de préférence 1 mm, par un séparateur en spirale (36) situé en amont et est tamisée au moyen d'un tamis vibrant (37) et la fraction limoneuse (39) à séparer ensuite est préconcentrée via le passage du tamis.
